(19) Europäisches Patentamt
European Patent Office
Office européen des brevets

(11) EP 4 210 118 A1

# (12) EUROPEAN PATENT APPLICATION
published in accordance with Art. 153(4) EPC

(43) Date of publication:
**12.07.2023 Bulletin 2023/28**

(21) Application number: **21864343.5**

(22) Date of filing: **01.09.2021**

(51) International Patent Classification (IPC):
***H01L 51/30*** (2006.01) ***C07D 471/22*** (2006.01)
***H01L 29/786*** (2006.01) ***H01L 51/05*** (2006.01)
***H01L 51/46*** (2006.01)

(52) Cooperative Patent Classification (CPC):
**C07D 471/22; H01L 29/786; H10K 10/00; H10K 85/00**

(86) International application number:
**PCT/JP2021/032043**

(87) International publication number:
**WO 2022/050286 (10.03.2022 Gazette 2022/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.09.2020 JP 2020147355**

(71) Applicants:
- **The University of Tokyo**
  **Bunkyo-ku, Tokyo 113-8654 (JP)**
- **Daicel Corporation**
  **Osaka 530-0011 (JP)**

(72) Inventors:
- **OKAMOTO, Toshihiro**
  **Tokyo 113-8654 (JP)**
- **KUROSAWA, Tadanori**
  **Tokyo 113-8654 (JP)**
- **YU, Craig Peiqi**
  **Tokyo 113-8654 (JP)**
- **TAKEYA, Junichi**
  **Tokyo 113-8654 (JP)**
- **YAMAMOTO, Akito**
  **Tokyo 108-8230 (JP)**
- **IKEDA, Daiji**
  **Tokyo 108-8230 (JP)**

(74) Representative: **Grünecker Patent- und Rechtsanwälte**
**PartG mbB**
**Leopoldstraße 4**
**80802 München (DE)**

## (54) ORGANIC SEMICONDUCTOR MATERIAL

(57) Provided is a compound capable of forming through a printing process an organic semiconductor exhibiting high charge mobility. The compound according to the present disclosure is represented by Formula (1) below. In Formula (1), $X^1$ and $X^2$ are the same or different and each represent -O-, -$NR^1$-, or -$PR^2$-. $Y^1$ and $Y^2$ are the same or different and each represent an oxygen atom or a sulfur atom. $Z^1$ to $Z^8$ are the same or different and each represent =Nor =$CR^3$-. The $R^1$, $R^2$, and $R^3$ are the same or different and each represent a hydrogen atom or an organic group. n represents an integer of 0 or greater.

EP 4 210 118 A1

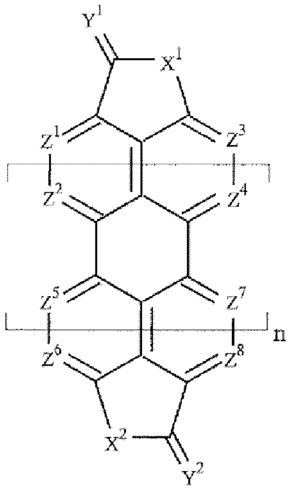

(1)

## Description

### Technical Field

The present disclosure relates to a novel organic semiconductor material. The present disclosure claims priority from the Japanese Patent Application No. 2020-147355 filed in Japan on September 2, 2020, the content of which is incorporated herein by reference.

### Background Art

Semiconductor devices such as transistors are used in devices with in integrated circuits (e.g., displays, RFIDs, and memory). As semiconductors constituting the semiconductor devices, inorganic semiconductors using raw materials such as silicon have been used.

Inorganic semiconductors exhibit extremely high charge mobility, but they have issues of being heavy and not deformable due to little flexibility. Also, because the production of inorganic semiconductors involves a vacuum deposition method which includes a process of heating at a high temperature of 300 to 1000°C to melt, vaporize, or sublimate an inorganic semiconductor material such as silicon, there are issues such as a difficulty in making an area increase and high costs.

Thus, in recent years, organic semiconductors that are light-weighted, flexible, and can be produced under mild conditions by using a printing process (i.e., a method in which an ink with an organic semiconductor material dissolved is applied to a substrate or the like) are attracting attention. Patent Document 1 discloses thionated perylene bisimide as a semiconductor material constituting an n-type organic semiconductor. Patent Document 2 discloses a compound represented by the following formula:

[Chem. 3]

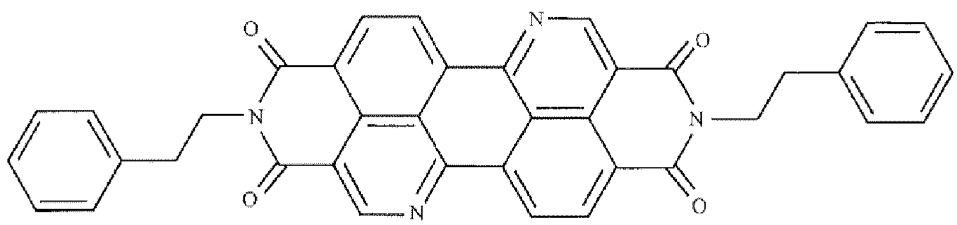

### Citation List

### Patent Document

Patent Document 1: WO 2011/082234

Patent Document 2: JP 2019-178160 A

### Summary of Invention

### Technical Problem

In an inorganic semiconductor in which atoms are bonded through covalent bonding, charge transport occurs through the covalent bonding. In contrast, in an organic semiconductor, charge transport occurs through weak overlapping of molecular orbitals. Therefore, an organic semiconductor has lower charge mobility compared to an inorganic semiconductor.

Furthermore, because the compounds described in Patent Documents 1 and 2 have low solvent solubilities, a thin film crystal having a large domain size cannot be formed by printing process (or solution process). Therefore, there is an issue of further reduction in charge mobility that was originally low.

An object of the present disclosure is to provide an organic semiconductor material that can form an organic

semiconductor exhibiting high charge mobility by a printing process.

Another object of the present disclosure is to provide an organic semiconductor composition that can form an organic semiconductor exhibiting high charge mobility by a printing process.

Another object of the present disclosure is to provide an organic semiconductor film exhibiting high charge mobility and a production method thereof.

Another object of the present disclosure is to provide a device including an organic semiconductor film exhibiting high charge mobility and a production method thereof.

Solution to Problem

As a result of diligent research to solve the issues described above, the present inventors found the following.

1. The compound represented by Formula (1) below (hereinafter, also referred to as "compound (1)") has excellent solvent solubility and can form a thin film crystal having a large domain size by a printing process.
2. The organic semiconductor containing the compound (1) has a herringbone crystal structure and has high charge mobility due to a large overlapping of molecular orbitals.

The present disclosure has been completed based on these findings.

Specifically, the present disclosure provides a compound represented by the following Formula (1):

[Chem. 1]

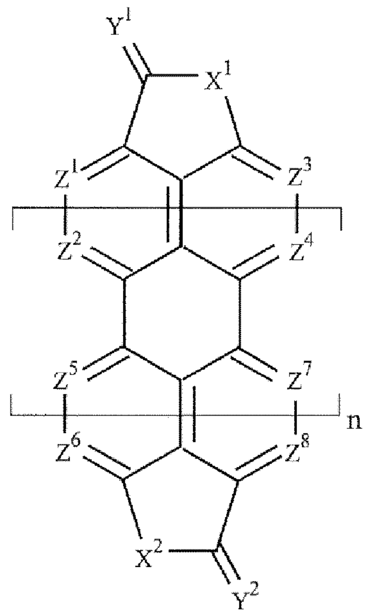

(1)

where in Formula (1), $X^1$ and $X^2$ are the same or different and each represent - O-, $-NR^1-$, or $-PR^2-$; $Y^1$ and $Y^2$ are the same or different and each represent an oxygen atom or a sulfur atom; $Z^1$ to $Z^8$ are the same or different and each represent =N- or $=CR^3-$; The $R^1$, $R^2$, and $R^3$ are the same or different and each represent a hydrogen atom or an organic group; and n represents an integer of 0 or greater.

The present disclosure also provides an organic semiconductor composition containing the compound represented by Formula (1) and a solvent.

The present disclosure also provides a method for producing an organic semiconductor film. The method includes applying a solvent-dissolved material containing the compound represented by Formula (1) on a substrate and drying to form a film containing the compound.

The present disclosure also provides a method for producing an organic semiconductor device. The method includes applying a solvent-dissolved material of the compound represented by Formula (1) on a substrate and drying to form a film containing the compound.

The present disclosure also provides an organic semiconductor film containing the compound represented by the following Formula (1):

[Chem. 2]

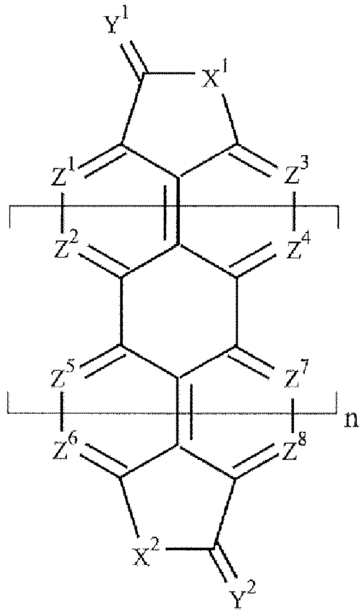

(1)

where in Formula (1), $X^1$ and $X^2$ are the same or different and each represent - O-, $-NR^1-$, or $-PR^2-$; $Y^1$ and $Y^2$ are the same or different and each represent an oxygen atom or a sulfur atom; $Z^1$ to $Z^8$ are the same or different and each represent =N- or $=CR^3-$; The $R^1$, $R^2$, and $R^3$ are the same or different and each represent a hydrogen atom or an organic group; and n represents an integer of 0 or greater.

The present disclosure also provides an organic semiconductor device including the organic semiconductor film.

Advantageous Effects of Invention

The compound (1) has excellent solvent solubility. Through a printing process, a thin film having a homogeneous film properties can be formed, and thus an organic semiconductor having a large domain size can be produced. By using the compound (1) having the characteristics described above, making an area increase of an organic semiconductor is achieved easily.

The organic semiconductor (n-type organic semiconductor) containing the compound (1) is light-weighted and has flexibility. And it has high charge mobility.

By using the compound (1), an organic semiconductor device (e.g., organic thin film transistors, organic thin film solar cells, and photoelectric conversion elements) that is thin and light-weighted and has flexibility, high conductivity, and high stability (stability against heat, water, electricity, and the like) can be produced through a printing process.

Description of Embodiments

Compound 1

The compound (1) according to the present disclosure is a compound represented by Formula (1) below:

[Chem. 4]

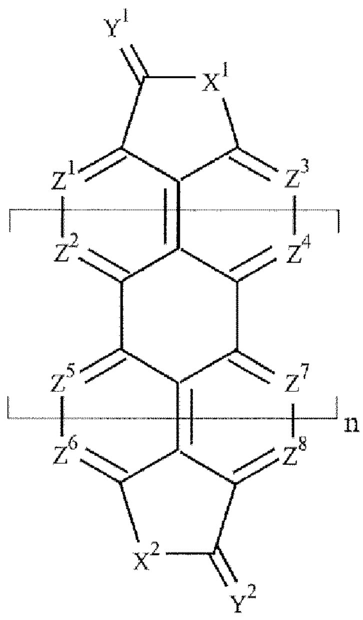

(1)

where in Formula (1), $X^1$ and $X^2$ are the same or different and each represent - O-, -$NR^1$-, or -$PR^2$-; $Y^1$ and $Y^2$ are the same or different and each represent an oxygen atom or a sulfur atom; $Z^1$ to $Z^8$ are the same or different and each represent =N- or =$CR^3$-; The $R^1$, $R^2$, and $R^3$ are the same or different and each represent a hydrogen atom or an organic group; and n represents an integer of 0 or greater.

Examples of the organic groups of the $R^1$, $R^2$, and $R^3$ include monovalent hydrocarbon groups, monovalent halogenated hydrocarbon groups, monovalent heterocyclic groups, silyl groups, cyano groups, halogen atoms, hydroxy groups, substituted oxy groups (e.g., alkoxy groups, aryloxy groups, aralkyloxy groups, and acyloxy groups), carboxyl groups, cyano groups, nitro groups, substituted or unsubstituted amino groups, sulfo groups, phosphonic acid groups, and monovalent groups each obtained by bonding two or more of these through a single bond or a linking group. Furthermore, the hydroxy group and the carboxyl group may be protected by a protective group that is commonly used in the field of organic synthesis.

Examples of the linking group include a $C_{1-5}$ alkylene group, a carbonyl group (-CO-), an ether bond (-O-), a thioether bond (-S-), an ester bond (-COO-), an amide bond (-CONH-), and a carbonate bond (-OCOO-).

The hydrocarbon group includes aliphatic hydrocarbon groups, alicyclic hydrocarbon groups, and aromatic hydrocarbon groups.

Examples of the aliphatic hydrocarbon group include alkyl groups having from 1 to 20 (preferably from 1 to 10, and particularly preferably from 1 to 6) carbons, such as a methyl group, an ethyl group, a propyl group, an isopropyl group, a butyl group, an isobutyl group, an s-butyl group, a t-butyl group, a pentyl group, a hexyl group, a decyl group, and a dodecyl group; alkenyl groups having from 2 to 20 (preferably from 2 to 10, and particularly preferably from 2 to 3) carbons, such as a vinyl group, an allyl group, and a 1-butenyl group; and alkynyl groups having from 2 to 20 (preferably from 2 to 10, and particularly preferably from 2 to 3) carbons, such as an ethynyl group and a propynyl group.

Examples of the alicyclic hydrocarbon group include 3- to 20-membered (preferably 3- to 15-membered, and particularly preferably 5- to 8-membered) cycloalkyl groups, such as a cyclopropyl group, a cyclobutyl group, a cyclopentyl group, a cyclohexyl group, and a cycloheptyl group, and a cyclooctyl group; 3- to 20-membered (preferably 3- to 15-membered, and particularly preferably 5- to 8-membered) cycloalkenyl groups, such as a cyclopentenyl group and a cyclohexenyl group; and bridged cyclic hydrocarbon groups, such as a perhydronaphthalen-1-yl group, a norbornyl group, an adamantyl group, a tricyclo[5.2.1.$0^{2,6}$]decan-8-yl group, and a tetracyclo[4.4.0.$1^{2,5}$.$1^{7,10}$]dodecan-3-yl group.

Examples of the aromatic hydrocarbon group include aryl groups having from 6 to 20 (preferably from 6 to 14, and particularly preferably from 6 to 10) carbons, such as a phenyl group and a naphthyl group; and aralkyl groups having from 7 to 15 carbons, such as a phenylethyl group.

Examples of the halogenated hydrocarbon group include those in which at least one of hydrogen atoms contained in the hydrocarbon group described above is replaced with at least one of halogen atoms selected from the group consisting of a fluorine atom, a chlorine atom, a bromine atom, and an iodine atom.

The monovalent heterocyclic group has the heterocycle structure with one hydrogen atom removed. The heterocycles include aromatic heterocycles and non-aromatic heterocycles. Examples of such a heterocycle include 3- to

20-membered rings (preferably 3- to 10-membered rings, and particularly preferably 4- to 6-membered rings) having carbon atoms and at least one heteroatom (e.g., oxygen atom, sulfur atom, or nitrogen atom) as atoms constituting the ring, and condensed rings thereof. Specific examples thereof include heterocycles containing an oxygen atom as a heteroatom (e.g., 3-membered rings, such as an oxirane ring; 4-membered rings, such as an oxetane ring; 5-membered rings, such as a furan ring, a tetrahydrofuran ring, an oxazole ring, an isoxazole ring, and a γ-butyrolactone ring; 6-membered rings, such as a 4-oxo-4H-pyran ring, a tetrahydropyran ring, and a morpholine ring; condensed rings, such as a benzofuran ring, an isobenzofuran ring, a 4-oxo-4H-chromene ring, a chroman ring, and an isochroman ring; crosslinked rings, such as a 3-oxatricyclo[4.3. $1.1^{4,8}$]undecan-2-one ring and a 3-oxatricyclo[$4.2.1.0^{4,8}$]nonan-2-one ring), heterocycles containing a sulfur atom as a heteroatom (e.g., 5-membered rings, such as a thiophene ring, a thiazole ring, an isothiazole ring, and a thiadiazole ring; and 6-membered rings, such as a 4-oxo-4H-thiopyran ring; condensed rings, such as a benzothiophene ring), and heterocycles containing a nitrogen atom as a heteroatom (e.g., 5-membered rings, such as a pyrrole ring, a pyrrolidine ring, a pyrazole ring, an imidazole ring, and a triazole ring; 6-membered rings, such as an isocyanuric ring, a pyridine ring, a pyridazine ring, a pyrimidine ring, a pyrazine ring, a piperidine ring, and a piperazine ring; condensed rings, such as an indole ring, an indoline ring, a quinoline ring, an acridine ring, a naphthyridine ring, a quinazoline ring, and a purine ring).

The silyl group is a group represented by $-Si(R)_3$, and the three Rs are the same or different and each represent a hydrocarbon group.

The hydrocarbon group in the R takes the same examples as those for the hydrocarbon groups in $R^1$, $R^2$, and $R^3$ described above. Among these, the R is preferably an alkyl group having from 1 to 10 carbons or an aryl group having from 6 to 10 carbons.

Examples of the silyl group include a trimethylsilyl group, a triisopropylsilyl group, a triphenylsilyl group, and a dimethylphenylsilyl group.

For the $R^1$ and $R^2$, a monovalent hydrocarbon group, a monovalent halogenated hydrocarbon group, or a monovalent heterocyclic group is preferred, and a monovalent aliphatic hydrocarbon group, a monovalent aromatic hydrocarbon group (particularly an aryl group), a group in which at least one hydrogen atom contained in the hydrocarbon group is replaced with halogen atom(s), or a monovalent aromatic heterocyclic group is more preferred.

For the $R^1$ and $R^2$, from the viewpoint of excellent solvent solubility, a monovalent hydrocarbon group is preferred, a monovalent aliphatic hydrocarbon group is more preferred, and a monovalent saturated aliphatic hydrocarbon group is particularly preferred. The number of carbons in the group is preferably from 1 to 20, particularly preferably from 1 to 12, most preferably from 4 to 12, and especially preferably from 5 to 10.

For the $R^1$ and $R^2$, from the viewpoint of excellent solvent solubility, an alkyl group is preferred. The alkyl group include linear alkyl groups and branched alkyl groups. Among these, a linear alkyl group is preferred. The number of carbons in the alkyl group is preferably from 1 to 20, particularly preferably from 1 to 12, most preferably from 4 to 12, and especially preferably from 5 to 10.

For the $R^1$ and $R^2$, from the viewpoint of achieving excellent solvent solubility and exhibiting high charge mobility by facilitating two-dimensional charge transfer because an effective mass ratio of electrons is small and overlapping of molecular orbitals is large, a monovalent halogenated hydrocarbon group (i.e., a group in which at least one of hydrogen atoms contained in the monovalent hydrocarbon group is replaced with halogen atom(s)) is preferred. Furthermore, among the halogenated hydrocarbon groups, a monovalent halogenated aliphatic hydrocarbon group is preferred, a monovalent halogenated saturated aliphatic hydrocarbon group is more preferred, an alkyl halide group is particularly preferred, and a linear alkyl halide group is most preferred. The number of carbons in the monovalent halogenated hydrocarbon group is preferably from 1 to 20, more preferably from 1 to 12, particularly preferably from 2 to 10, most preferably from 2 to 8, and especially preferably from 2 to 6.

The alkyl halide group is, for example, represented by chemical formula $[C_sX_tH_{(2s+1-t)}]$. In this chemical formula, s represents an integer from 1 to 20, preferably an integer from 1 to 12, particularly preferably an integer from 2 to 10, most preferably an integer from 2 to 8, and especially preferably an integer from 2 to 6. t represents an integer of 1 or greater. The upper limit of t is 2s + 1. X represents a halogen atom.

The $R^3$ is preferably a hydrogen atom, a cyano group, or a trialkylsilylethynyl group. The alkyl group in the trialkylsilylethynyl group is, for example, an alkyl group having from 1 to 4 carbons.

A compound represented by Formula (1), in which $Z^4$ and $Z^5$ in the formula represent =C(CN)-, is preferred from the viewpoint of achieving excellent solvent solubility. Furthermore, the compound represented by Formula (1), in which $Z^3$ and $Z^6$ in the formula represent =C(CN)-, is preferred from the viewpoints of achieving excellent solvent solubility and exhibiting high charge mobility by facilitating two-dimensional charge transfer due to a brickwork structure formed by the cyano groups in $Z^3$ and $Z^6$.

n is a number of the repeating unit represented inside the parentheses of Formula (1) and represents an integer of 0 or greater. The upper limit for the value of n is, for example, 5, preferably 4, particularly preferably 3, and most preferably 2. The lower limit for the value of n is preferably 1.

The $Y^1$ and $Y^2$ are each an oxygen atom or a sulfur atom, which is appropriately selected based on capability

of reducing a difference of LUMO of the compound (1) and work function of electrode material and thus reducing threshold voltage. For example, in a case where gold, silver, or the like is used as an electrode material, a sulfur atom is preferred.

For the compound represented by Formula (1) above, compounds represented by the following Formulas (1-0) to (1-3) are preferred, a compound represented by the following Formula (1-1) or (1-2) is particularly preferred, and a compound represented by the following Formula (1-1) is most preferred. In the following formulas, $X^1$, $X^2$, $Y^1$, $Y^2$, and $Z^1$ to $Z^8$ are the same as those described above. $Z^9$ to $Z^{16}$ are the same or different and each represent =N- or =$CR^3$-, and $R^3$ represents a hydrogen atom or an organic group.

[Chem. 5]

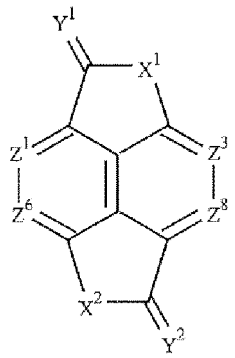

(1-0)

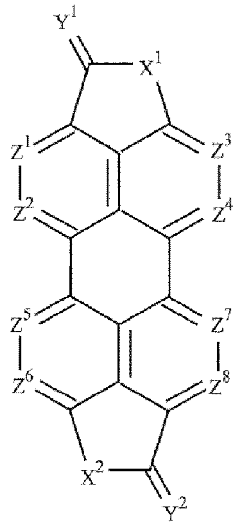

(1-1)

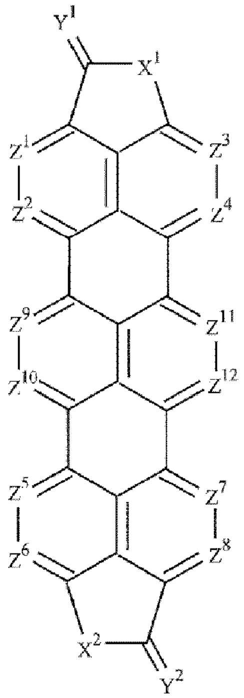

(1-2)

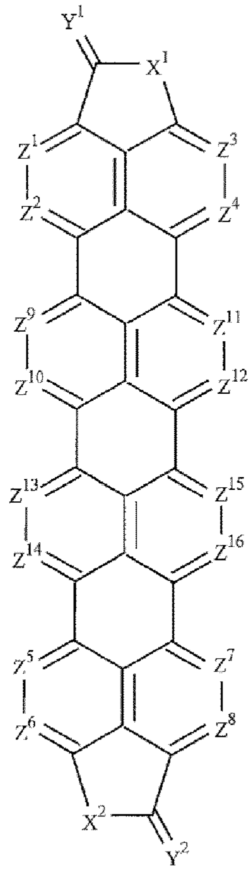

(1-3)

Furthermore, in a case where n in Formula (1) above is 0, that is in a case of the compound represented by Formula (1-0) above, the $Z^6$ preferably represents =N-, and $Z^1$, $Z^3$, and $Z^8$ each preferably represent =$CR^3$-.

In a case where n in Formula (1) above is 1, that is in a case of the compound represented by Formula (1-1) above, the $Z^2$ and $Z^7$ each preferably represent =N-, $Z^1$, $Z^3$, $Z^4$, $Z^5$, $Z^6$, and $Z^8$ each preferably represent =$CR^3$-.

In a case where n in Formula (1) above is 2, that is in a case of the compound represented by Formula (1-2) above, the $Z^2$, $Z^5$, and $Z^{11}$ each preferably represent =N-, and $Z^1$, $Z^3$, $Z^4$, $Z^6$, $Z^7$, $Z^8$, $Z^9$, $Z^{10}$, and $Z^{12}$ each preferably represent =$CR^3$-.

In the case of the compound represented by Formula (1-2) above, the $Z^2$, $Z^5$, and $Z^{12}$ may each represent =N-, and $Z^1$, $Z^3$, $Z^4$, $Z^6$, $Z^7$, $Z^8$, $Z^9$, $Z^{10}$, and $Z^{11}$ may each represent =$CR^3$-.

In the case of the compound represented by Formula (1-2) above, the $Z^2$, $Z^7$, and $Z^{10}$ may each represent =N-, and $Z^1$, $Z^3$, $Z^4$, $Z^5$, $Z^6$, $Z^8$, $Z^9$, $Z^{11}$, and $Z^{12}$ may each represent =$CR^3$-.

In the case of the compound represented by Formula (1-2) above, the $Z^2$ and $Z^7$ may each represent =N-, and $Z^1$, $Z^3$, $Z^4$, $Z^5$, $Z^6$, $Z^8$, $Z^9$, $Z^{10}$, $Z^{11}$, and $Z^{12}$ may each represent =$CR^3$-.

In a case where n in Formula (1) above is 3, that is in a case of the compound represented by Formula (1-3) above, the $Z^2$, $Z^7$, $Z^{11}$, and $Z^{14}$ each preferably represent =N-, and $Z^1$, $Z^3$, $Z^4$, $Z^5$, $Z^6$, $Z^8$, $Z^9$, $Z^{10}$, $Z^{12}$, $Z^{13}$, $Z^{15}$, and $Z^{16}$ each preferably represent =$CR^3$-.

In the case of the compound represented by Formula (1-3) above, the $Z^2$ and $Z^7$ may each represent =N-, and

$Z^1$, $Z^3$, $Z^4$, $Z^5$, $Z^6$, $Z^8$, $Z^9$, $Z^{10}$, $Z^{11}$, $Z^{12}$, $Z^{13}$, $Z^{14}$, $Z^{15}$, and $Z^{16}$ may each represent $=CR^3-$.

For the compound represented by Formula (1) above, a compound (1-1-1), which is represented by Formula (1-1) above in which $Z^2$ and $Z^7$ each represent =N-, $Z^4$ and $Z^5$ each represent =C(CN)-, and $Z^1$, $Z^3$, $Z^6$, and $Z^8$ each represent =CH-, is preferred from the viewpoint of achieving excellent solvent solubility. Furthermore, a compound (1-1-2), which is represented by Formula (1-1) above in which $Z^2$ and $Z^7$ each represent =N-, $Z^3$ and $Z^6$ each represent =C(CN)-, and $Z^1$, $Z^4$, $Z^5$, and $Z^8$ each represent =CH-, is preferred from the viewpoints of achieving excellent solvent solubility and exhibiting high charge mobility by facilitating two-dimensional charge transfer due to a brickwork structure formed by the cyano groups.

The compound represented by Formula (1) above is preferably a compound represented by the following Formula (2). In the formula below, $X^1$, $X^2$, $Y^1$, $Y^2$, and n are the same as those described above.

[Chem. 6]

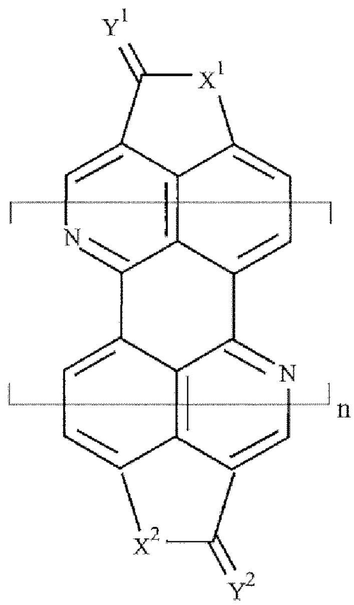

(2)

As the compound represented by Formula (2) above, compounds represented by the following Formulas (2-0), (2-1), (2-2a) to (2-2c) and (2-3) are preferred, compounds represented by the following Formulas (2-1), (2-2a), (2-2b), and (2-2c) are particularly preferred, and a compound represented by the following Formula (2-1) is most preferred. In the formulas below, $X^1$, $X^2$, $Y^1$, and $Y^2$ are the same as those described above.

[Chem. 7]

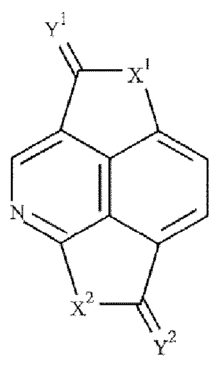

(2-0)

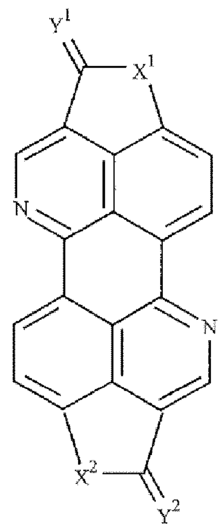

(2-1)

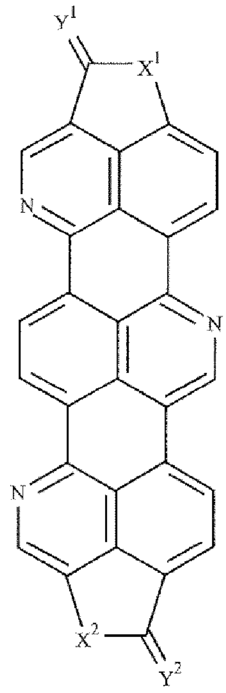

(2-2a)

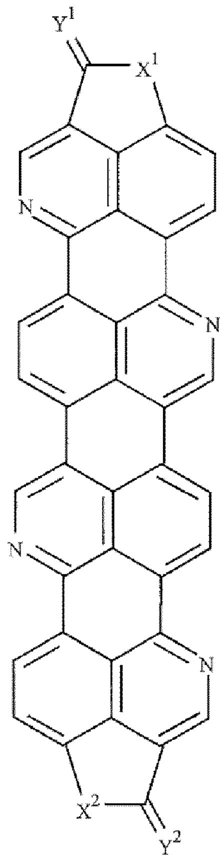

(2-3)

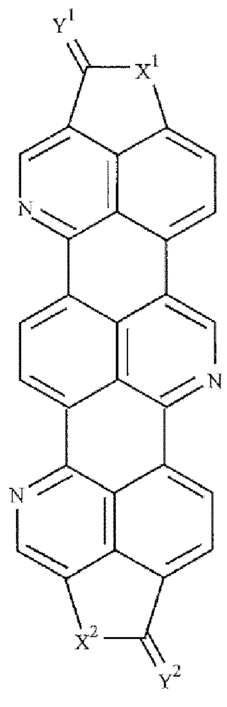

(2-2b)

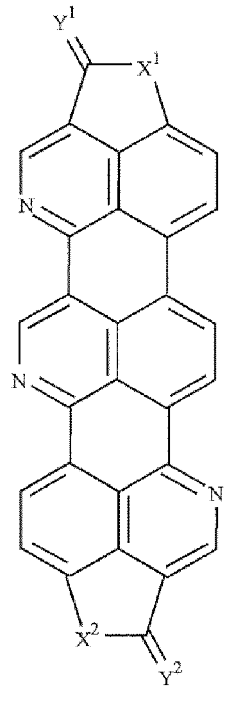

(2-2c)

The $X^1$ and $X^2$ are the same or different and each represent -O-, $-NR^1-$, or $-PR^2-$. Among these, $X^1$ and $X^2$ are preferably the same and are each preferably a group represented by $-NR^1-$.

The compound represented by Formula (2) above is preferably a compound represented by the following Formula (2-1-1) from the viewpoint of achieving excellent solvent solubility. Furthermore, the compound represented by Formula (2-1-2) below is preferred from the viewpoint of achieving excellent solvent solubility and exhibiting high charge mobility by increasing transfer integral between molecular orbitals, which relates to electron transfer, (e.g., increasing to 40 meV or greater, preferably increasing to 50 meV or greater, and particularly preferably increasing to 60 meV or greater) by facilitating two-dimensional charge transfer due to a brickwork structure formed by the cyano groups. Note that, in the following formulas, $X^1$, $X^2$, $Y^1$, and $Y^2$ are the same as those described above.

[Chem. 8]

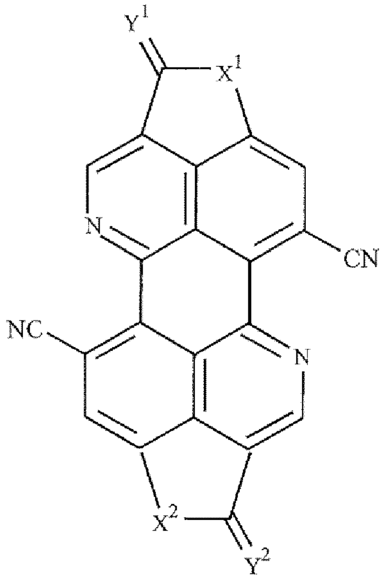

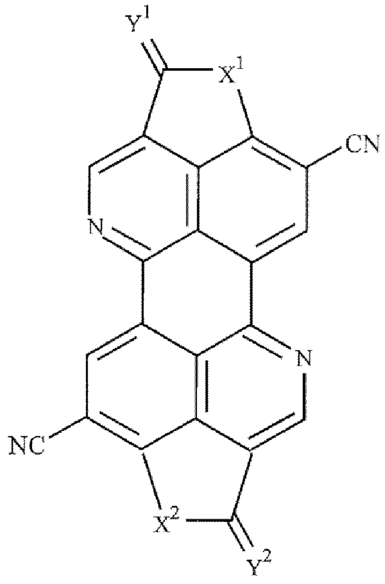

(2-1-1) (2-1-2)

The compound represented by Formula (1) above is preferably a compound represented by the following Formula (3). In the formula below, $Y^1$, $Y^2$, $R^1$, $Z^1$ to $Z^8$, and n are the same as those described above.

[Chem. 9]

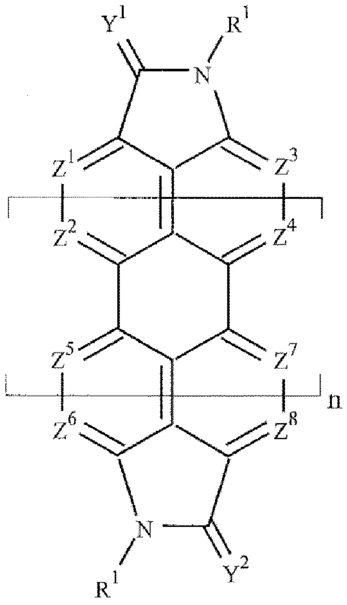

(3)

For the compound represented by Formula (3) above, compounds represented by Formulas (3-0) to (3-3) below are preferred, a compound represented by Formula (3-1) or (3-2) below is particularly preferred, and a compound represented by Formula (3-1) below is most preferred. In the formulas below, $Y^1$, $Y^2$, $R^1$, and $Z^1$ to $Z^8$ are the same as those described above.

[Chem. 10]

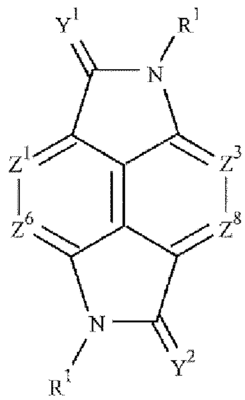

(3-0)

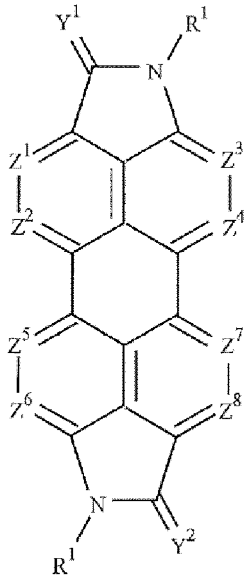

(3-1)

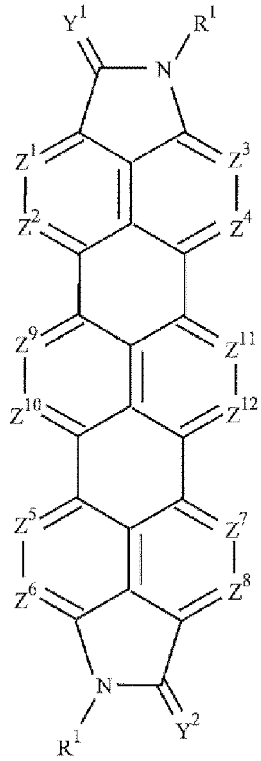

(3-2)

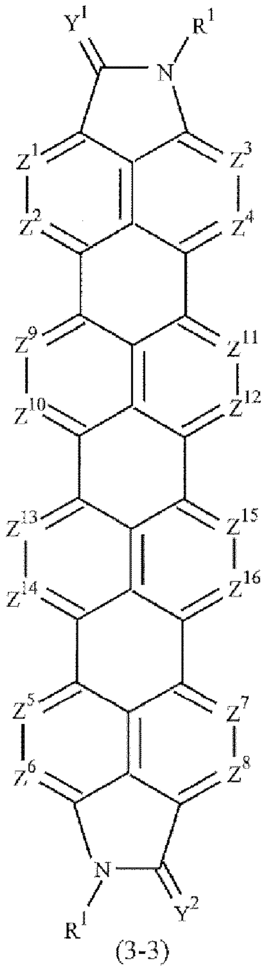

(3-3)

Furthermore, in a case where n in Formula (3) above is 0, that is in a case of the compound represented by Formula (3-0) above, the $Z^6$ preferably represents =N-, and $Z^1$, $Z^3$, and $Z^8$ each preferably represent $=CR^3$-.

In a case where n in Formula (3) above is 1, that is in a case of the compound represented by Formula (3-1) above, the $Z^2$ and $Z^7$ each preferably represent =N-, $Z^1$, $Z^3$, $Z^4$, $Z^5$, $Z^6$, and $Z^8$ each preferably represent $=CR^3$-.

In a case where n in Formula (3) above is 2, that is in a case of the compound represented by Formula (3-2) above, the $Z^2$, $Z^5$, and $Z^{11}$ each preferably represent =N-, and $Z^1$, $Z^3$, $Z^4$, $Z^6$, $Z^7$, $Z^8$, $Z^9$, $Z^{10}$, and $Z^{12}$ each preferably represent $=CR^3$-.

In the case of the compound represented by Formula (3-2) above, the $Z^2$, $Z^5$, and $Z^{12}$ may each represent =N-, and $Z^1$, $Z^3$, $Z^4$, $Z^6$, $Z^7$, $Z^8$, $Z^9$, $Z^{10}$, and $Z^{11}$ may each represent $=CR^3$-.

In the case of the compound represented by Formula (3-2) above, the $Z^2$, $Z^7$, and $Z^{10}$ may each represent =N-, and $Z^1$, $Z^3$, $Z^4$, $Z^5$, $Z^6$, $Z^8$, $Z^9$, $Z^{11}$, and $Z^{12}$ may each represent $=CR^3$-.

In the case of the compound represented by Formula (3-2) above, the $Z^2$ and $Z^7$ may each represent =N-, and $Z^1$, $Z^3$, $Z^4$, $Z^5$, $Z^6$, $Z^8$, $Z^9$, $Z^{10}$, $Z^{11}$, and $Z^{12}$ may each represent $=CR^3$-.

In a case where n in Formula (3) above is 3, that is in a case of the compound represented by Formula (3-3) above, the $Z^2$, $Z^7$, $Z^{11}$, and $Z^{14}$ each preferably represent =N-, and $Z^1$, $Z^3$, $Z^4$, $Z^5$, $Z^6$, $Z^8$, $Z^9$, $Z^{10}$, $Z^{12}$, $Z^{13}$, $Z^{15}$, and $Z^{16}$ each preferably represent $=CR^3$-.

In the case of the compound represented by Formula (3-3) above, the $Z^2$ and $Z^7$ may each represent =N-, and $Z^1$, $Z^3$, $Z^4$, $Z^5$, $Z^6$, $Z^8$, $Z^9$, $Z^{10}$, $Z^{11}$, $Z^{12}$, $Z^{13}$, $Z^{14}$, $Z^{15}$, and $Z^{16}$ may each represent $=CR^3$-.

As the compound represented by Formula (3) above, a compound represented by Formula (3-1) above, in which $Z^2$ and $Z^7$ each represent =N-, $Z^4$ and $Z^5$ each represent =C(CN)-, and $Z^1$, $Z^3$, $Z^6$, and $Z^8$ each represent =CH-, is preferred from the viewpoint of achieving excellent solvent solubility. Furthermore, a compound represented by Formula (3-1) above, in which $Z^2$ and $Z^7$ each represent =N-, $Z^3$ and $Z^6$ each represent =C(CN)-, and $Z^1$, $Z^4$, $Z^5$, and $Z^8$ each represent =CH-, is preferred from the viewpoints of achieving excellent solvent solubility and exhibiting high charge

mobility by facilitating two-dimensional charge transfer due to a brickwork structure formed by the cyano groups in $Z^3$ and $Z^6$.

The compound represented by Formula (1) above is preferably a compound represented by Formula (4) below. In the formula below, $Y^1$, $Y^2$, $R^1$, and n are the same as those described above.

[Chem. 11]

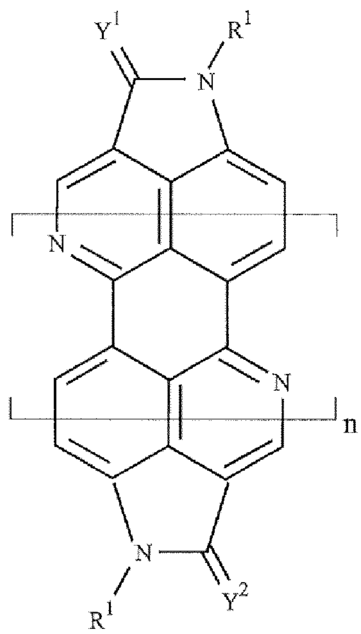

(4)

For the compound represented by Formula (4) above, compounds represented by Formulas (4-0), (4-1), (4-2a) to (4-2c) and (4-3) below are preferred, compounds represented by Formulas (4-1), (4-2a), (4-2b), and (4-2c) below are particularly preferred, and a compound represented by Formula (4-1) below is most preferred. In the formulas below, $Y^1$, $Y^2$, and $R^1$ are the same as those described above.

[Chem. 12]

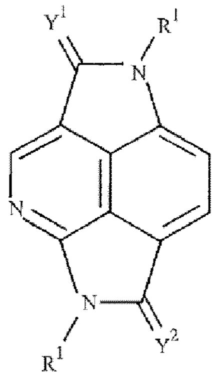

(4-0)

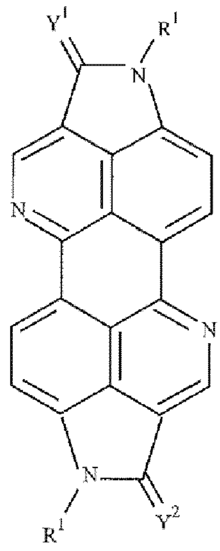

(4-1)

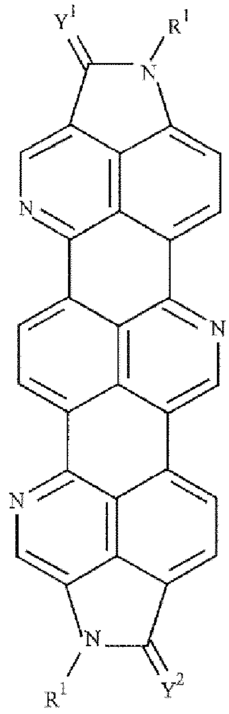

(4-2a)

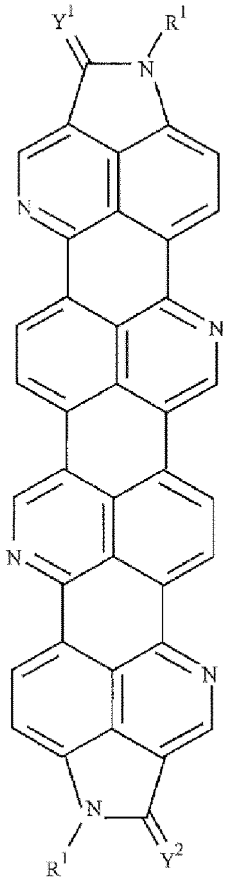

(4-3)

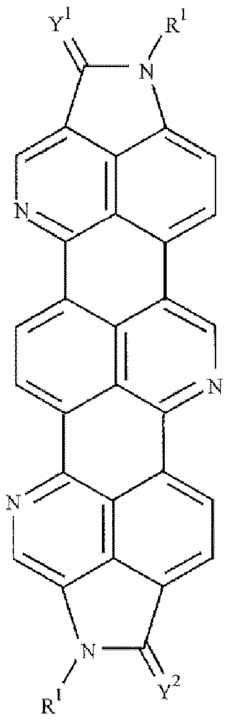

(4-2b)

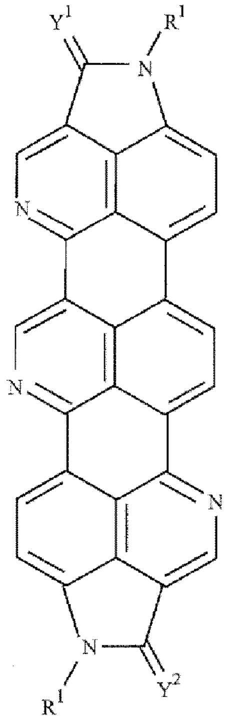

(4-2c)

The compound represented by Formula (4) above is preferably a compound represented by Formula (4-1-1) below from the viewpoint of achieving excellent solvent solubility. Furthermore, a compound represented by Formula (4-1-2) below is preferred from the viewpoints of achieving excellent solvent solubility and exhibiting high charge mobility by facilitating two-dimensional charge transfer due to a brickwork structure formed by the cyano groups in the formula below. Note that, in the formulas below, $Y^1$, $Y^2$, and $R^1$ are the same as those described above.

[Chem. 13]

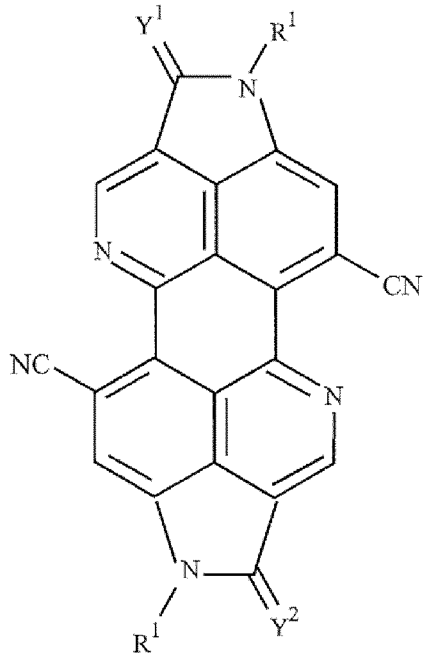

(4-1-1)

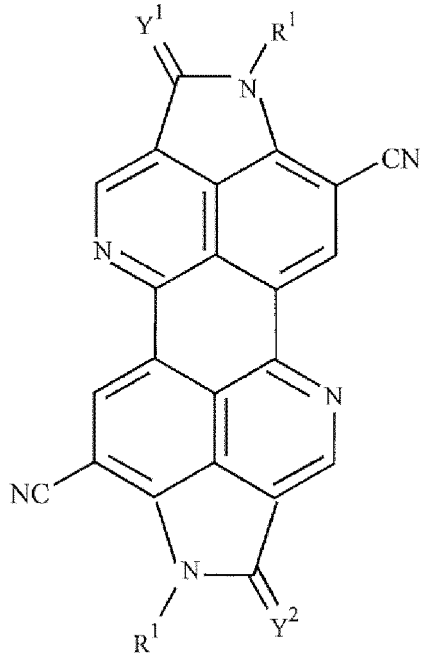

(4-1-2)

The compound (1) above has larger polarization compared to the compound(s) disclosed in Patent Document 2. Thus, the solvent solubility is excellent. When a solvent-dissolved material of the compound (1) is used, even more uniform film can be formed by a printing process.

Furthermore, the compound (1) has less carbonyl groups and less hydrogen bonds between molecules formed by the carbonyl groups compared to the compound disclosed in Patent Document 2. While a film containing the compound described in Patent Document 2 has a brickwork crystal structure, a film containing the compound (1) has a herringbone crystal structure. In the herringbone crystal structure, an overlapping of molecular orbitals is larger than that in the brickwork crystal structure. Thus, the compound (1) exhibits higher charge mobility than the compound(s) disclosed in Patent Document 2. Accordingly, a film containing the compound (1) exhibits high charge mobility.

Because the compound (1) has the properties described above, the compound (1) is useful as a raw material for forming an organic semiconductor (i.e., organic semiconductor material) and, particularly, extremely useful as a raw material for forming an organic semiconductor by a printing process.

The compound represented by Formula (4) above, in which $Y^1$ and $Y^2$ in the formula are each an oxygen atom, namely a compound represented by Formula (4’) below (hereinafter, also referred to as "compound (4’)")) can be produced by, for example, reacting a compound represented by Formula (4’-1) below (hereinafter, also referred to as "compound (4’-1)") with $R^1$-$NH_2$.

[Chem. 14]

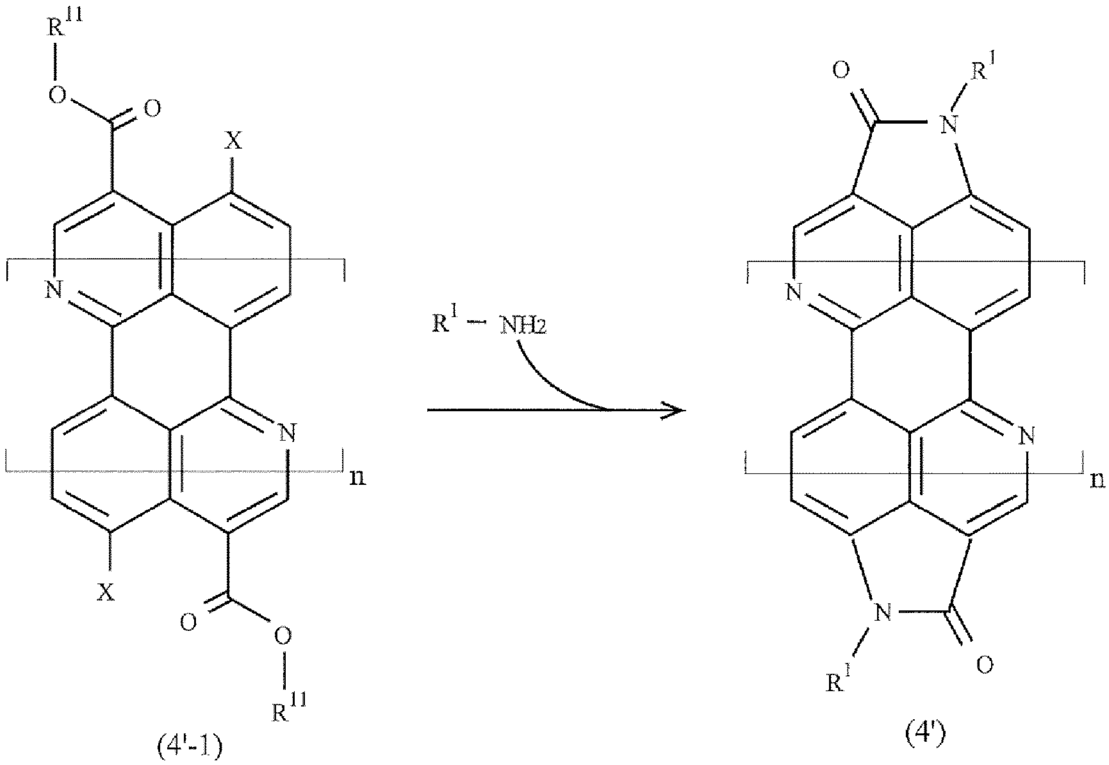


(4'-1)

(4')

$R^1$ and n in Formulas (4'-1) and (4') above are the same as those described above. $R^{11}$ represents an alkyl group having from 1 to 5 carbon(s) or an aryl group having from 6 to 10 carbons. In the alkyl group and the aryl group, one or two or more of hydrogen atoms contained in each of the groups may be replaced with halogen atom(s). That is, it may be an alkyl halide group or a halogenated aryl group (e.g., trichlorophenyl group).

The amount of $R^1$-$NH_2$ to be used is, for example, from 2 to 10 mol per 1 mol of the compound (4'-1).

The above reaction may be performed in the presence of a base. Examples of the foregoing base include 1,8-diazabicyclo[5.4.0]undecene-7 (DBU) or salts thereof (e.g., a phenol salt, an octylate, a p-toluene sulfonate, a formate, and a tetraphenylborate salt); 1,5-diazabicyclo[4.3.0]nonene-5 (DBN) or salts thereof (e.g., a phenol salt, an octylate, a p-toluene sulfonate, a formate, and a tetraphenylborate salt).

The amount of dehydrohalogenation agent to be used is, for example, from 0.2 to 2 mol per 1 mol of the compound (4'-1).

The reaction may be performed in the presence of a solvent. Examples of the solvent include benzene and derivatives thereof (the benzene derivatives are each a compound in which one or two or more hydrogen atoms contained in the benzene are replaced with group(s) selected from the group consisting of halogen atoms, alkyl groups, haloalkyl groups, alkoxy groups, cyano groups, nitro groups, and substituted oxycarbonyl groups) such as benzene, toluene, xylene, ethylbenzene, trifluoromethylbenzene, trifluorotoluene, chlorobenzene, dichlorobenzene, anisole, benzonitrile, nitrobenzene, and ethyl benzoate; aliphatic hydrocarbons such as hexane, heptane, and octane; alicyclic hydrocarbons such as cyclohexane; haloalkanes such as carbon tetrachloride, chloroform, dichloromethane, and 1,2-dichloroethane; ketones such as acetone and methyl ethyl ketone; amides such as N,N-dimethylformamide and N,N-dimethylacetamide; nitriles such as acetonitrile and propionitrile; and chain or cyclic ethers such as diethyl ether, dibutyl ether, dimethoxyethane, dioxane, and tetrahydrofuran. One of these can be used alone or two or more in combination.

The amount of the solvent to be used is an amount that makes the concentration of the compound (4'-1) becomes, for example, approximately from 0.01 to 0.2 mol/L. The solvent, when used in an amount greater than the above range, decreases concentrations of the reaction components, and tends to decrease the reaction rate.

With the progress of the reaction, the alcohol produced as a by-product in the reaction system may be captured by a known dealcoholization agent (e.g., molecular sieve).

A reaction atmosphere may be any atmosphere that does not inhibit the reaction and is not particularly limited, and may be, for example, any of an air atmosphere, a nitrogen atmosphere, and an argon atmosphere.

The reaction temperature is, for example, from 120 to 180°C. The reaction time is, for example, from 0.5 to 10 hours. In addition, the reaction can be carried out by any method, such as a batch method, a semi-batch method, and a continuous method.

After completion of the reaction, the resulting reaction product can be separated and purified by a separation means, such as filtration, concentration, distillation, extraction, crystallization, adsorption, recrystallization, column chromatography, or sublimation; or a separation means in combination of these.

The compound represented by Formula (4) above, in which $Y^1$ and $Y^2$ in the formula are each a sulfur atom, namely a compound represented by Formula (4") below (hereinafter, also referred to as "compound (4")") can be produced by, for example, reacting the compound (4') with a sulfurizing agent. $R^1$ and n in the formulas below are the same as those described above.

[Chem. 15]

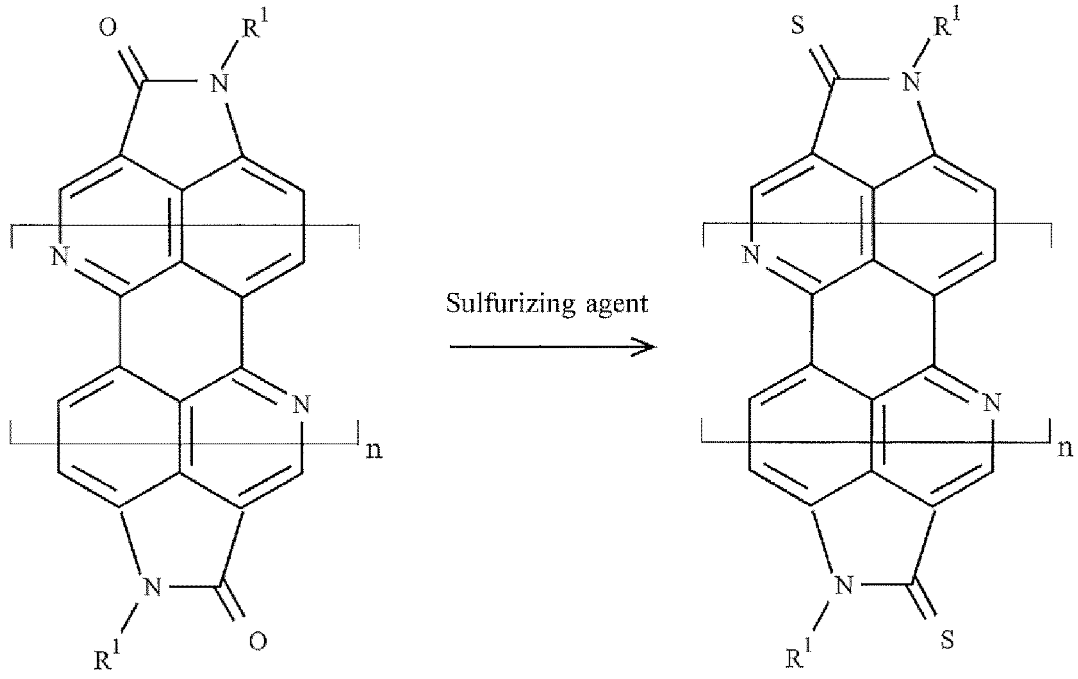


(4') (4")

Examples of the sulfurizing agent include Lawesson's reagent.

The amount of the sulfurizing agent to be used is, for example, from 2 to 10 mol per 1 mol of the compound (4').

The reaction may be performed in the presence of a solvent. Examples of the solvent include benzene and derivatives thereof such as benzene, toluene, xylene, ethylbenzene, trifluoromethylbenzene, trifluorotoluene, chlorobenzene, dichlorobenzene, anisole, benzonitrile, nitrobenzene, and ethyl benzoate (the benzene derivative means a compound in which one hydrogen or two or more hydrogen atoms contained in the benzene are replaced with group(s) selected from the group consisting of a halogen atom, an alkyl group, a haloalkyl group, an alkoxy group, a cyano group, a nitro group, and a substituted oxycarbonyl group); aliphatic hydrocarbons such as hexane, heptane, and octane; alicyclic hydrocarbons such as cyclohexane; haloalkanes such as carbon tetrachloride, chloroform, dichloromethane, and 1,2-dichloroethane; ketones such as acetone and methyl ethyl ketone; esters such as methyl acetate, ethyl acetate, isopropyl acetate, and butyl acetate; amides such as N,N-dimethylformamide and N,N-dimethylacetamide; nitriles such as acetonitrile and propionitrile; chain or cyclic ethers such as diethyl ether, dibutyl ether, dimethoxyethane, dioxane, and tetrahydrofuran; and organic acids such as acetic acid. One of these can be used alone or two or more in combination.

The amount of the solvent to be used is an amount that makes the concentration of the compound (4') becomes, for example, approximately from 0.01 to 0.2 mol/L.

A reaction atmosphere may be any atmosphere that does not inhibit the reaction and is not particularly limited, and may be, for example, any of an air atmosphere, a nitrogen atmosphere, and an argon atmosphere.

The reaction temperature is, for example, from 90 to 180°C. The reaction time is, for example, from 0.5 to 70 hours. In addition, the reaction can be carried out by any method, such as a batch method, a semi-batch method, and a continuous method.

After completion of the reaction, the resulting reaction product can be separated and purified by a separation means, such as filtration, concentration, distillation, extraction, crystallization, adsorption, recrystallization, or column chromatography; or a separation means in combination of these.

The compound represented by Formula (4) above, in which $R^1$ in the formula is an alkyl halide group, (i.e., compound represented by the following Formula (6)) can be produced by, for example, reacting the compound (5) with a base and then reacting a haloalkyl triflate. In the formulas below, n, s, t, and X are the same as those described above.

[Chem. 16]

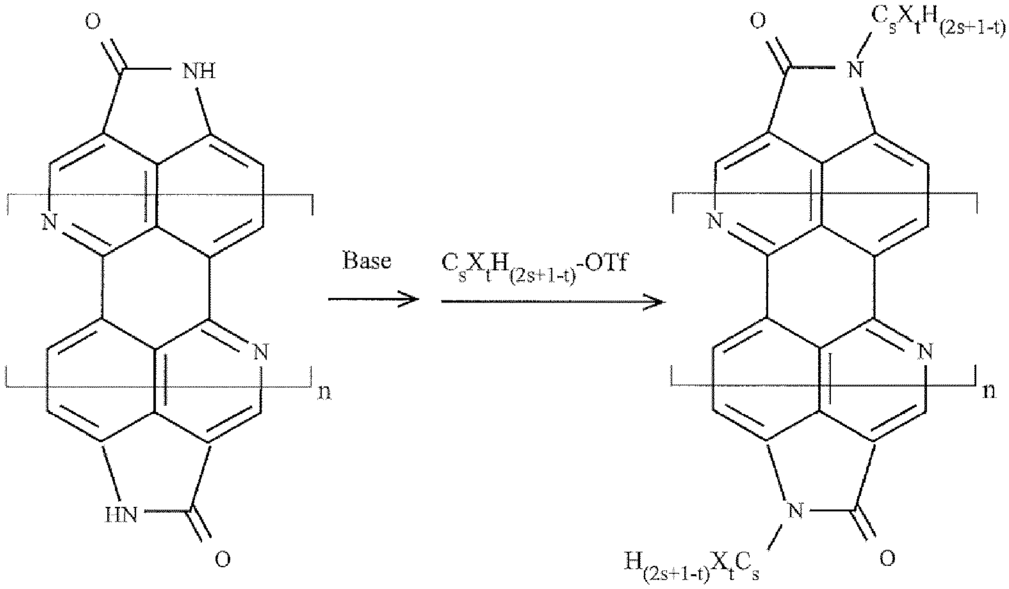


(5) (6)

Examples of the base include organic bases such as triethylamine, diisopropylethylamine, pyridine, 2,6-lutidine, and diazabicycloundecene; and inorganic bases such as sodium carbonate, potassium carbonate, cesium carbonate, sodium hydroxide, potassium hydroxide, sodium hydride, and potassium hydride. One of these can be used alone or two or more in combination.

The amount of the base to be used is, for example, from 1 to 5 mol per 1 mol of the compound (5).

The reaction of the compound (5) and the base can be performed in the presence of a solvent. For the solvent, for example, a polar aprotic solvent, such as dimethyl sulfoxide, N,N-dimethylformamide, and N-methylpyrrolidone, can be used. Furthermore, the reaction is preferably performed in a temperature range that is not lower than room temperature and not higher than the boiling point of the solvent.

The amount of the haloalkyl triflate to be used is, for example, from 10 to 30 mol per 1 mol of the compound (5).

The reaction of the compound (5) and the haloalkyl triflate can be performed in the presence of a solvent. For the solvent, a solvent same as the solvent that can be used in the reaction of the compound (5) and the base can be used. Furthermore, the reaction temperature is, for example, from 70 to 130°C, and the reaction time is, for example, from 0.5 to 24 hours. The reaction can be carried out by a batch method, a semi-batch method, or a continuous method.

After completion of the reaction, the resulting reaction product can be separated and purified by a separation means, such as filtration, concentration, distillation, extraction, crystallization, adsorption, recrystallization, or column chromatography; or a separation means in combination of these.

Among the compound (4'-1), a compound in which n in the formula is 1 (i.e., compound represented by Formula (4'-7) below) can be produced through the reactions [1] to [5] below.

[Chem. 17]

[1]

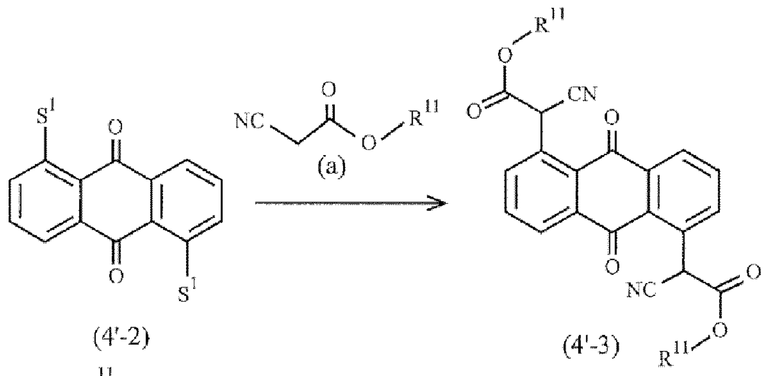

(a)

(4'-2) (4'-3)

[2]

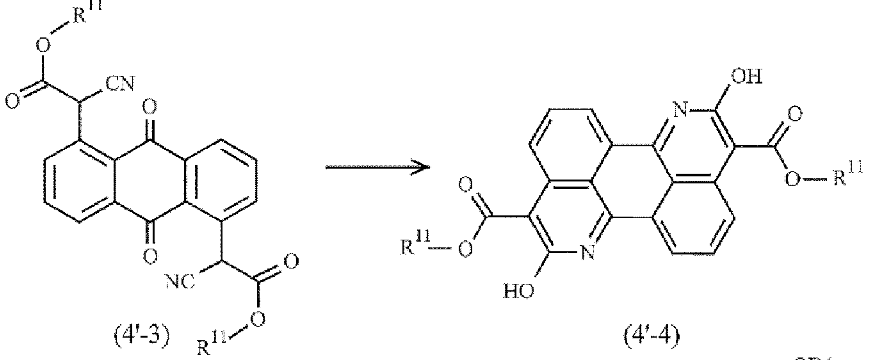

(4'-3) (4'-4)

[3]

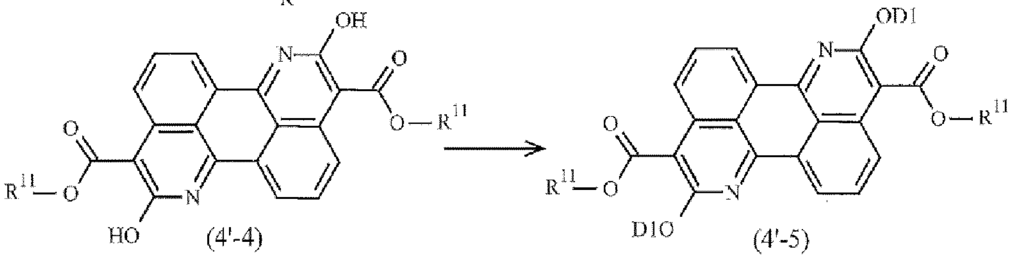

(4'-4) (4'-5)

[4]

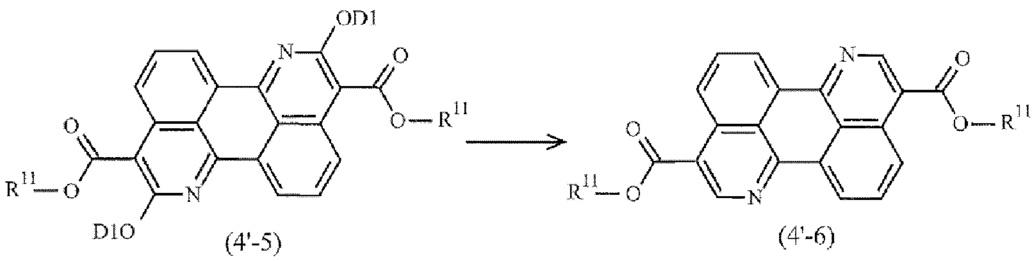

(4'-5) (4'-6)

[5]

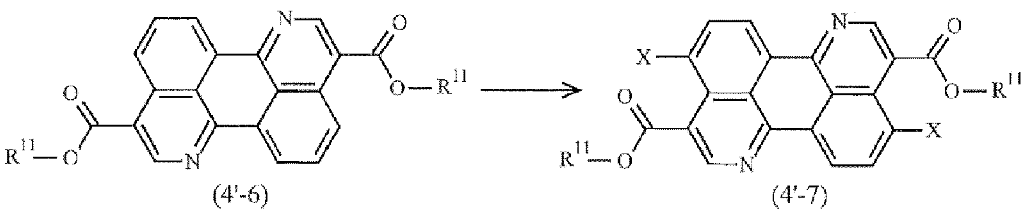

(4'-6) (4'-7)

In the formulas above, $S^1$ represents an electron-withdrawing group. Examples of the electron-withdrawing group include $-NH^{3+}$, $-CF_3$, $-CCl_3$, $-NO_2$, -CN, -CHO, $-COCH_3$, $-COOC_2H_5$, -COOH, $-SO_2CH_3$, and $-SO_3H$.

$R^{11}$ in the formulas above is as described above.

In the formulas, D1 represents a leaving group. Examples of the leaving group include $-OSO_2CF_3$, which is -OTf, and $-OSO_2F$.

In the formulas above, X represents a halogen atom.

Reaction [1]

The reaction [1] is a reaction to obtain the compound represented by Formula (4'-3) above by reacting the compound represented by Formula (4'-2) above with the compound represented by Formula (a) above.

The amount of the compound represented by Formula (a) above to be used is, for example, from 2 to 10 mol per 1 mol of the compound represented by Formula (4'-2) above.

The reaction [1] is preferably performed in the presence of a base as a catalytic promoter. Examples of the base include inorganic bases and organic bases. Examples of the inorganic base include hydroxides, carbonates, and hydrogencarbonates of alkali metals (e.g., sodium and potassium); and hydroxides and carbonates of alkaline earth metals (e.g., magnesium and calcium). Examples of the organic base include quaternary ammonium salts such as tetramethylammonium hydroxide; alkoxides such as sodium ethoxide and potassium t-butoxide; metal amides such as lithium diisopropylamide, potassium hexamethyldisilazide, and lithium-2,2,6,6-tetramethylpiperidide (LiTMP); metal alkyls such as alkyl lithium and alkyl aluminum; amines such as triethylamine and N,N-dimethylaniline; pyridine-based heterocyclic amines such as pyridine and DMAP; non-pyridine-based heterocyclic amines, piperidine, N-methylpiperidine, N-methylpyrrolidine, DBU, DBN, and imidazole; and phosphorus-based bases such as BEMP and guanidino phosphatase.

The amount of the base to be used is, for example, from 2 to 10 mol per 1 mol of the compound represented by Formula (4'-2) above.

The reaction temperature is, for example, from 40 to 90°C. The reaction time is, for example, from 0.5 to 10 hours.

The reaction may be performed in the presence of a solvent. As the solvent, for example, a sulfoxide-based solvent such as dimethyl sulfoxide can be used.

Reaction [2]

The reaction [2] is a reaction to obtain the compound represented by Formula (4'-4) above from the compound represented by Formula (4'-3) above.

In the reaction, first, water is added to a nitrile group of the compound represented by Formula (4'-3) above, and thus an imidic acid group is formed. This reaction is performed in the presence of an acid catalyst (e.g., sulfuric acid). Then, by dehydrating between the imidic acid group and an adjacent carbonyl group, the compound represented by Formula (4'-4) above is obtained. This reaction is performed in the presence of a dehydrating agent such as a strong base (e.g., sodium hydroxide).

Reaction [3]

The reaction [3] is a reaction to obtain the compound represented by Formula (4'-5) above by providing a leaving group to a hydroxy group of the compound represented by Formula (4'-4) above.

The compound represented by Formula (4'-5) above, in which the leaving group D1 is Tf, can be produced by reacting the compound represented by Formula (4'-4) above with N-phenylbis(trifluoromethanesulfonimide). The amount of the N-phenylbis(trifluoromethanesulfonimide) to be used is, for example, from 2 to 5 mol per 1 mol of the compound represented by Formula (4'-4) above.

The reaction is preferably performed in the presence of a base. As the base, bases in the examples described above can be used. Furthermore, the reaction may be performed in the presence of a solvent (e.g., haloalkane such as dichloromethane).

The reaction temperature is, for example, from -40 to 30°C. The reaction time is, for example, from 0.5 to 10 hours.

Reaction [4]

The reaction [4] is a reaction to obtain the compound represented by Formula (4'-6) above by removing the OD1 group of the compound represented by Formula (4'-5) above.

The reaction is preferably performed in the presence of a palladium catalyst (e.g., tetrakis(triphenylphosphine)palladium(0)).

The reaction is also preferably performed in the presence of a base. For the base, bases in the examples described above can be used.

The reaction is preferably performed in the presence of a solvent (e.g., formic acid or dimethylformamide)

Reaction [5]

The reaction [5] is a reaction to obtain the compound represented by Formula (4'-7) above by reacting the

compound represented by Formula (4'-6) above with a halogenating agent.

In a case where X in the compound represented by Formula (4'-7) above is a bromine atom, for the halogenating agent, for example, a brominating agent such as N-bromosuccinimide (NBS) can be used.

The amount of the halogenating agent to be used is, for example, from 2 to 10 mol per 1 mol of the compound represented by Formula (4'-6) above.

The reaction is preferably performed in the presence of concentrated sulfuric acid.

Organic semiconductor composition

The organic semiconductor composition of the present disclosure contains the compound (1) and a solvent. The organic semiconductor composition is ink to be used to form an organic semiconductor film by a printing process (in more detail, to form an organic semiconductor film by applying to a substrate and drying). Thus, the organic semiconductor composition is, for example, ink for organic semiconductor film formation.

For the solvent, any solvent that dissolves the compound (1) above may be used, and examples thereof include hydrocarbons such as hexane, octane, decane, toluene, xylene, mesitylene, ethylbenzene, amylbenzene, decalin, 1-methylnaphthalene, 1-ethylnaphthalene, 1,6-dimethylnaphthalene, and tetralin; ketones such as acetone, methyl ethyl ketone, methyl isobutyl ketone, cyclohexanone, acetophenone, propiophenone, and butyrophenone; halogenated hydrocarbons such as dichloromethane, chloroform, tetrachloromethane, dichloroethane, trichloroethane, tetrachloroethane, chlorobenzene, 1,2-dichlorobenzene, 1,2,4-trichlorobenzene, chlorotoluene, 1-chloronaphthalene, and 1-fluoronaphthalene; heterocycles such as pyridine, picoline, quinoline, thiophene, 3-butylthiophene, and thieno[2,3-b]thiophene; halogenated heterocycles such as chlorothiophene, dichlorothiophene, bromothiophene, dibromothiophene, and 3,4-dichloro-1,2,5-thiadiazole; esters such as ethyl acetate, butyl acetate, amyl acetate, 2-ethylhexyl acetate, γ-butyrolactone, and phenyl acetate; alcohols such as methanol, propanol, butanol, pentanol, hexanol, cyclohexanol, methyl cellosolve, ethyl cellosolve, and ethylene glycol; ethers such as dibutyl ether, tetrahydrofuran, dioxane, dimethoxyethane, anisole, ethoxybenzene, propoxybenzene, isopropoxybenzene, butoxybenzene, methylanisole, dimethylanisole, 1,4-benzodioxane, and phenoxytoluene; amide or imides such as N,N-dimethylformamide, N,N-dimethylacetamide, 1-methyl-2-pyrrolidone, 1-methyl-2-imidazolidinone, and 1,3-dimethyl-2-imidazolidinone; sulfoxides such as dimethyl sulfoxide; phosphates such as trimethyl phosphate; and nitriles such as acetonitrile and benzonitrile. One of these can be used alone or two or more in combination.

For the solvent, from the viewpoints of being capable of suppressing volatilization of the solvent at the stage of application on a substrate, being capable of maintaining the composition concentration fixed by this, and being capable of maintaining excellent application properties, a solvent having a boiling point at normal pressure of 60°C or higher (preferably 80°C or higher, and particularly 100°C or higher) is preferred.

From the viewpoint of facilitating drying after the application, the boiling point of the solvent at normal pressure is preferably 160°C or lower, more preferably 140°C or lower, and particularly preferably 120°C or lower.

The content of the solvent is, for example, from 100 to 10000 parts by weight per 1 part by weight of the compound (1) above.

Furthermore, the content of the compound (1) is, for example, 50 wt.% or greater, preferably 60 wt.% or greater, particularly preferably 70 wt.% or greater, and most preferably 80 wt.% or greater, per the total non-volatile content in the organic semiconductor composition.

Furthermore, the total content of the compound (1) and the solvent is, for example, 50 wt.% or greater, preferably 60 wt.% or greater, particularly preferably 70 wt.% or greater, most preferably 80 wt.% or greater, and especially preferably 90 wt.% or greater, per the total amount of the organic semiconductor composition.

The organic semiconductor composition may contain one, or two or more of other components besides the compound (1) and the solvent. Examples of those other components include binder resins, surfactants, antioxidants, crystallization control agents, and crystalline orientation control agents. The blended amount of those other components (total amount in the case where two or more types are contained) is, for example, 50 wt.% or less, preferably 40 wt.% or less, particularly preferably 30 wt.% or less, most preferably 20 wt.% or less, and especially preferably 10 wt.% or less, per the total amount of the organic semiconductor composition.

The organic semiconductor composition can be prepared by blending the compound (1), the solvent, and other components as needed and appropriately agitating the mixture. The agitation may be performed while heating at a temperature that is lower than the boiling point of the solvent (e.g., approximately from 60 to 150°C).

Organic semiconductor film and production method thereof

The organic semiconductor film of the present disclosure contains the compound (1) described above.

The organic semiconductor film contains the compound (1). The compound (1) forms a herringbone crystal structure in the film. The organic semiconductor film thus exhibits high charge mobility, and therefore, it can be suitably

used as an organic semiconductor device material and an electrode material for a secondary battery.

The charge mobility of the organic semiconductor film is, for example, $10^{-5}$ $cm^2/Vs$ or greater, preferably $10^{-3}$ $cm^2/Vs$ or greater, particularly preferably $10^{-2}$ $cm^2/Vs$ or greater, and especially preferably.

The organic semiconductor film can be produced by either method of printing process or vapor deposition process; however, production by a printing process is preferred from the viewpoints of making an area increase easy and enabling to form an organic semiconductor film on a substrate with low heat resistance for high temperature conditions are not required. Furthermore, because the compound (1) has excellent solvent solubility, an organic semiconductor film exhibiting high charge mobility can be produced even by a printing process.

The method for producing an organic semiconductor film by a printing process includes forming a film containing the compound described above by applying a solvent-dissolved material of the compound (1) on a substrate and drying.

Examples of the solvent include the same examples as the solvent contained in the organic semiconductor composition described above. As the solvent-dissolved material of the compound (1), the organic semiconductor composition can be suitably used. The organic semiconductor film obtained by using the organic semiconductor composition includes a solidified material of the organic semiconductor composition.

Examples of the substrate include plastic substrates, silicon substrates, glass substrates, and ceramic substrates. Among these, a plastic substrate is preferred from the viewpoints of being light-weighted and having excellent flexibility.

The method of applying the solvent-dissolved material on the substrate is not particularly limited as long as the method can apply the solvent-dissolved material uniformly on the substrate. Printing methods such as a screen printing method, a mask printing method, an offset printing method, an inkjet printing method, a flexo printing method, a gravure printing method, a squeegee printing method, and a silkscreen printing method, are preferred because of ease and convenience and low cost.

The thickness of the applied solvent-dissolved material is, for example, from 10 to 100 nm.

Examples of a drying method for the solvent-dissolved material applied onto the substrate include a natural drying method at room temperature, vacuum drying, and drying by heating.

Organic semiconductor device and production method thereof

The organic semiconductor device according to the present disclosure at least include the organic semiconductor film.

Examples of the organic semiconductor device include switching elements, diodes, transistors, photoelectric conversion devices, solar cells, and photoelectric conversion elements (e.g., solar cell elements and organic electroluminescence elements).

The organic semiconductor device can be produced by, for example, forming a film containing the compound described above by applying a solvent-dissolved material of the compound (1) on a substrate and drying. The process can be performed in the same manner as in the method for producing the organic semiconductor film.

The organic semiconductor device includes the film containing the compound (1) and thus has both high electrical conductivity and high stability (stability against heat, water, electricity, and the like). In addition, weight reduction, flexibility, thickness reduction, and area increase can be realized at low cost.

Each of the configurations, their combinations, and the like of the present disclosure above is an example, and addition, omission, substitution, and change of the configuration can be appropriately made without departing from the gist of the present disclosure. In addition, the present disclosure is not limited by the embodiments and is limited only by the claims.

Examples

Hereinafter, the present disclosure will be described more specifically with reference to examples, but the present disclosure is not limited by these examples.

All of the following reactions were performed under an argon atmosphere. A compound (1) represented by Formula (I) below (dimethyl 4,10-dibromobenzo[de]isoquinolino[1,8-gh]quinoline-3,9-dicarboxylate) was synthesized in accordance with the method described in Patent Document 2.

Example 1

With the compound (I) (300 mg, 0.568 mmol, 1.0 eq), 11.4 mL of o-dichlorobenzene, diazabicycloundecene (DBU, 42 μL, 0.285 mmol, 0.5 eq), n-octylamine (0.56 mL, 3.41 mmol, 6.0 eq), and molecular sieve 4A (1.80 g) were added and agitated at 150°C for 3 hours. Thereafter, the mixture was cooled to room temperature, the solvent was volatilized, purification was performed by a silica gel column chromatography, and thus a compound represented by

Formula (II) below, namely a compound (II) (127 mg; yield: 40%) was obtained. The structure was identified by $^1$H-NMR. (400 MHz,$CDCl_2CDCl_2$ at 100°C): δ 9.12 (s, 2H), 8.66 (d, J = 7.6 Hz, 2H), 7.05 (d, J = 7.6 Hz, 2H), 3.90 (t, J = 7.2 Hz, 4H), 1.82 (t, J = 7.2 Hz, 4H), 1.2-1.5 (m, 20H), 0.87 (t, J = 7.2 Hz, 6H).

[Chem. 18]

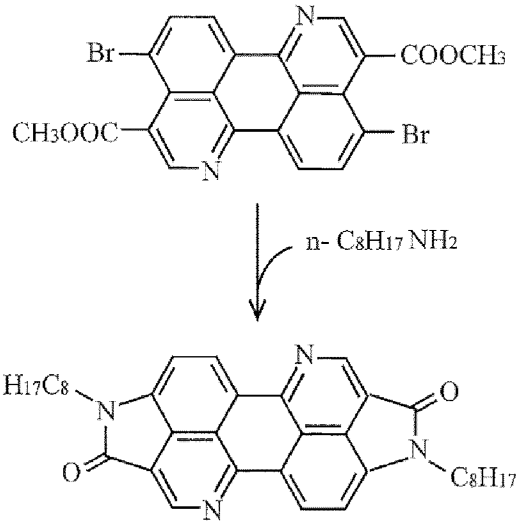


(I)

(II)

Example 2

With the compound (II) obtained by the same method as in Example 1 (56.1 mg, 0.10 mmol, 1.0 eq), 2 mL of toluene, and Lawesson's reagent (2,4-bis(4-methoxyphenyl)-1,3,2,4-dithiadiphosphetane 2,4-disulfide, 202 mg, 0.5 mmol, 5.0 eq) were added and agitated at reflux temperature for 40 hours. After the mixture was cooled to room temperature, the solvent was volatilized, purification was performed by a silica gel column chromatography, and thus a compound represented by Formula (III) below, namely a compound (III) (16 mg; yield: 27%) was obtained. The structure of the reaction product was identified by $^1$H-NMR.
(400 MHz, $CDCl_2CDCl_2$ at 100°C): δ 9.29 (s, 2H), 8.72 (d, J = 8.0 Hz, 2H), 7.24 (d, J = 8.0 Hz, 2H), 4.34 (t, J = 7.6 Hz, 4H), 1.90 (t, J = 7.6 Hz, 4H), 1.2 - 1.5 (m, 20H), 0.86 (t, J = 7.6 Hz, 6H).

[Chem. 19]

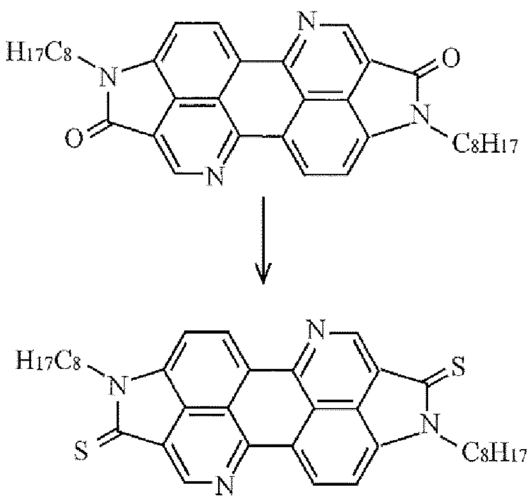

(II)

(III)

Example 3

A compound represented by Formula (IV) below, namely a compound (IV) was obtained in the same manner as in Example 1 except for using n-propylamine (0.280 mL, 3.41 mmol, 6.0 eq) in place of the n-octylamine.

[Chem. 20]

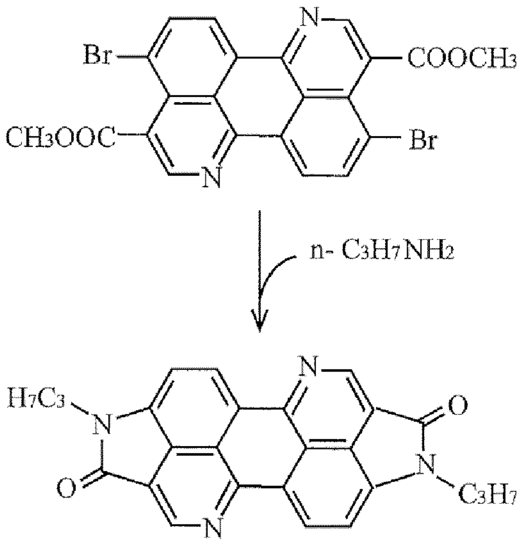


(I)

(IV)

In a Schlenk tube, the compound (IV) (113 mg, 0.269 mmol, 1.00 eq), N-bromosuccinimide (144 mg, 0.806 mmol, 3.00 eq), palladium(II) acetate (3.00 mg, 0.0134 mmol, 5 mol%), degassed acetic acid (50.0 μL, 0.806 mmol, 3.00 eq), 1,1,2,2-tetrachloroethane (7.70 mL), and N,N-dimethylformamide (1.30 mL) were added. The reaction mixture was agitated at 110°C for 1 hour and cooled to room temperature. Then, methanol was added to the reaction mixture and filtered. As a residue, a deep red solid was obtained (130 mg; yield: 95%). When the structure of the obtained solid was identified by $^{1}H$-NMR, it was confirmed that the solid was a compound represented by Formula (V) below, namely a compound (V).
(400 MHz, $CDCl_2CDCl_2$ at 100°C): δ 9.28 (s, 2H), 7.39 (s, 2H), 3.87 (t, J = 6.4 Hz, 4H), 1.87 (m, 4H), 1.04 (t, J = 7.2 Hz, 6H).

[Chem. 21]

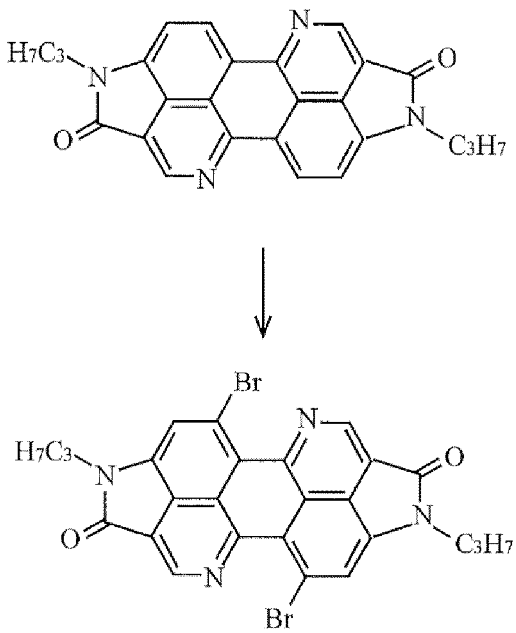

(IV)

(V)

Example 4

In a Schlenk tube, the compound (V) obtained by the same method as in Example 3 (100 mg, 0.173 mmol, 1.00 eq), copper cyanide (279 mg, 89.6 mmol, 18.0 eq) and N,N-dimethylformamide (7 mL) were added. The reaction mixture was agitated at 80°C for 1 hour and cooled to room temperature. Then, methanol was added to the reaction mixture and filtered. The obtained residue was dissolved in 200 mL o-dichlorobenzene and passed through a short column of Celite, and inorganic substances were removed. By removing the solvent under a reduced pressure, a deep purple solid was obtained (61 mg; yield: 75%). When the structure of the obtained solid was identified by $^1$H-NMR, it was confirmed that the solid was a compound represented by Formula (VI) below, namely a compound (VI).
(400 MHz, $CDCl_2CDCl_2$ at 100°C): δ 9.37 (s, 2H), 7.23 (s, 2H), 3.89 (t, J = 7.2 Hz, 4H), 1.86 (m, 4H), 1.05 (t, J = 7.2 Hz, 6H).

[Chem. 22]

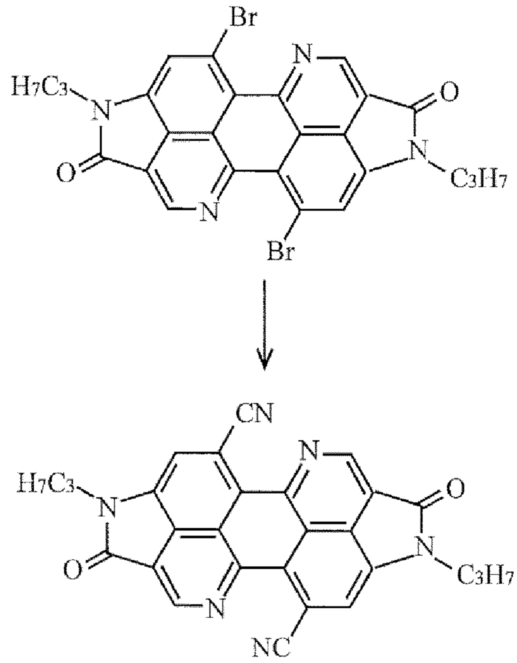

(V)

(VI)

Example 5

A compound represented by Formula (VII) below, namely a compound (VII) was obtained in the same manner as in Example 3 except for using n-hexylamine (0.448 mL, 3.41 mmol, 6.0 eq) in place of the n-propylamine.

A product was obtained in the same manner as in Example 4 except for using the compound (VII) in place of the compound (V). When the structure of the product was identified by $^1$H-NMR, it was confirmed that the product was a compound represented by Formula (VIII) below, namely a compound (VIII). The crystal structure of the compound (VIII) is a one-dimensional structure capable of one-dimensional conduction, and the transfer integral value of LUMO based on the crystal structure was 30 meV.
(400 MHz, $CDCl_2CDCl_2$ at 100°C): δ 9.36 (s, 2H), 7.23 (s, 2H), 3.91 (t, J = 7.2 Hz, 4H), 1.83 (m, 4H), 1.2-1.5 (m, 12H), 0.91 (t, J = 7.2 Hz, 6H).

[Chem. 23]

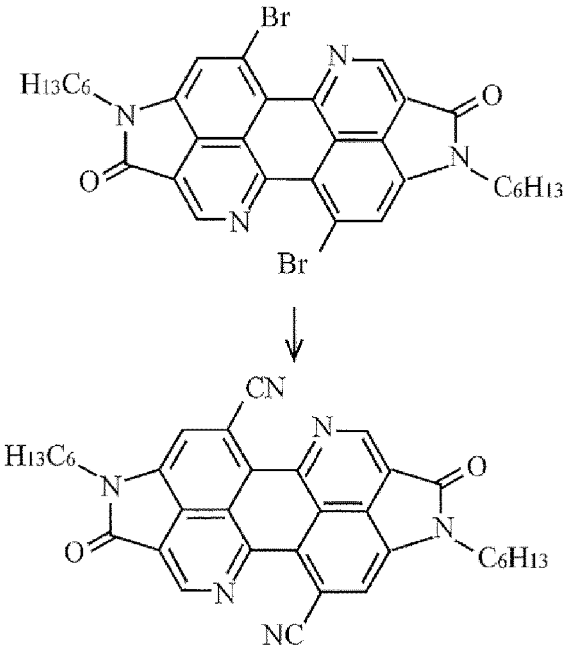

(VII)

(VIII)

Solvent solubility evaluation

For each of the compounds (II), (III), (VI), and (VIII) obtained in Examples 1, 2, 4, and 5 and a compound represented by Formula (IX) below, namely a compound (IX), as Comparative Example 1, and a compound represented by Formula (X) below, namely a compound (X), as Comparative Example 2, solvent solubility was evaluated by the following method.

[Chem. 24]

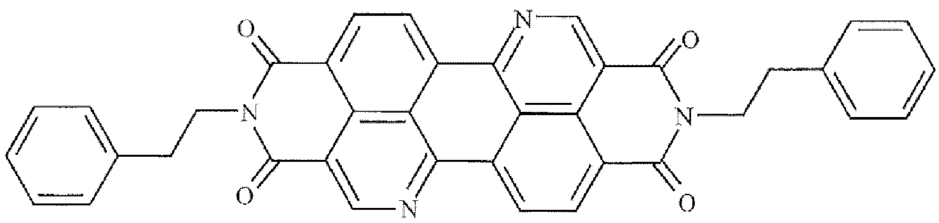

(IX)

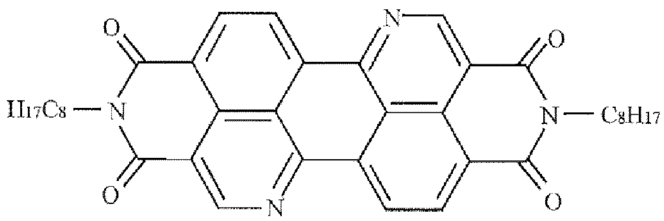

(X)

Evaluation method of solvent solubility

Approximately 1 mg of each compound (accurate weight was w (mg)) was weighed in a sample tube, and 20

μL of o-dichlorobenzene was added thereto by using a micropipette. The sample tube was capped and heated at 100°C by a hot plate. Whether or not the compound was dissolved was visually confirmed. When the compound was not dissolved, the operation of adding o-dichlorobenzene in an amount of 20 μL per addition until the compound was dissolved and heating to 100°C was repeated.

The solubility (wt.%) of the compound was calculated based on the following formula. Note that V in the formula is a volume (μL) of the o-dichlorobenzene added to dissolve the compound, and d is a density (g/$cm^3$) of the o-dichlorobenzene.

$$\text{Solubility (\%)} = (w/Vd) \times 100$$

The result is indicated in Table 1 below.

[Table 1]

Table 1

| | | Solubility (%) |
|---|---|---|
| Example 1 | Compound (II) | 0.58 |
| Example 2 | Compound (III) | 0.49 |
| Example 4 | Compound (VI) | 0.027 |
| Example 5 | Compound (VIII) | 0.52 |
| Comparative Example 1 | Compound (IX) | <0.0007 |
| Comparative Example 2 | Compound (X) | 0.019 |

Example 6

The compound (VI) obtained in Example 4 and anisole were agitated at 120°C in a nitrogen atmosphere for 2 hours, and thus an organic semiconductor composition (1) having a concentration of the compound (VI) of 0.05 wt.% was obtained.

A surface of a thermal oxide film of an n-type silicon substrate (20 mm × 20 mm, thickness: 0.4 mm) having a thermal oxide film (silicon oxide film) having a thickness of 200 nm on a surface thereof was subjected to ultraviolet-ozone cleaning, and treated with β-phenethyltrimethoxysilane.

A glass member (10 mm × 2 mm, thickness: 5 mm) was placed at the center of the β-phenethyltrimethoxysilane-treated surface of the substrate after the treatment in close contact therewith, and the substrate was heated to 60°C. Then, using a pipette, one drop (approximately 0.05 mL) of the organic semiconductor composition (1) was dropped from the side of the glass member to form a liquid film surrounding the glass member and having a concave meniscus.

Under normal pressure, the substrate was heated at 60°C for 2 hours, the obtained liquid membrane was dried, and thus crystal was deposited. The glass member was then removed, and heating and drying was performed under reduced pressure ($10^{-3}$ Pa) at 50°C for 12 hours, and thus a ring-like organic semiconductor film (1) having a uniform thickness (film thickness: 50 nm) was formed on the substrate.

The obtained organic semiconductor film (1) was masked, then gold was vapor-deposited (thickness: 40 nm), and thus an organic thin film transistor (1) for measuring field effect transistor properties [gate width (W): 300 μm; gate length (L): 50 μm; and a ratio (W/L): 6.0] was obtained.

Using a semiconductor parameter analyzer (4156C, available from Agilent) connected with a semi-auto prober (AX-2000, available from Vector Semiconductor Co., Ltd.) at normal pressure and at room temperature, a voltage of 70 V was applied between the source electrode and the drain electrode of the obtained organic thin film transistor (1), the gate voltage was changed in a range of from -20 V to 70 V, the charge mobility (μ: $cm^2$/Vs) was calculated based on the following formula using the drain current $I_d$ that changes depending on the change of the gate voltage. As a result, the charge mobility (μ) was $4.9 \times 10^{-5}$ $cm^2$/Vs.

[Math. 1]

$$\mu = \frac{2L \cdot Id}{W \cdot Ci \left( Vg - Vth \right)^2}$$

In the formula, L is the gate length, W is the gate width, Id is the drain current, $C_i$ is the capacitance per unit area of the gate insulating film, $V_g$ is the gate voltage, and $V_{th}$ is the threshold voltage.

Example 7

The compound (VIII) obtained in Example 5 and 3-phenoxytoluene were agitated at 90°C in a nitrogen atmosphere for 2 hours, and thus an organic semiconductor composition (2) having a concentration of the compound (VIII) of 0.03 wt.% was obtained.

A liquid membrane with concave meniscus was formed in the same manner as in Example 6 except for using the organic semiconductor composition (2) in place of the organic semiconductor composition (1).

Under normal pressure, the substrate was heated at 100°C for 2 hours, the obtained liquid membrane was dried, and thus crystal was deposited. The glass member was then removed, and heating and drying was performed under reduced pressure ($10^{-3}$ Pa) at 100°C for 12 hours, and thus a ring-like organic semiconductor film (2) having a uniform thickness (film thickness: 50 nm) was formed on the substrate.

The obtained organic semiconductor film (2) was masked, then gold was vapor-deposited (thickness: 40 nm), and thus an organic thin film transistor (2) for measuring field effect transistor properties [gate width (W): 30 μm; gate length (L): 50 μm; and a ratio (W/L): 0.6] was obtained. The threshold voltage of the obtained organic thin film transistor (2) was 40 V.

Example 8

A product was obtained by performing the following reaction, the obtained product was subjected to recrystallization using toluene and isopropanol, and thus a compound represented by Formula (XI) below, namely a compound (XI) was obtained (60 mg; yield: 54%). For the obtained compound, the structure was identified by $^1$H-NMR. The crystal structure of the compound (XI) was a brickwork structure capable of two-dimensional conduction. The transfer integral value of LUMO based on the crystal structure was 80 meV.
(400 MHz, $CDCl_2CDCl_2$ at 100°C): δ 9.20 (s, 2H), 8.68 (s, 2H), 4.12 (t, J = 7.6 Hz, 4H), 1.78 (t, J = 7.6 Hz, 4H), 1.3-1.5 (m, 12H), 0.84 (t, J = 7.2 Hz, 6H).

[Chem. 25]

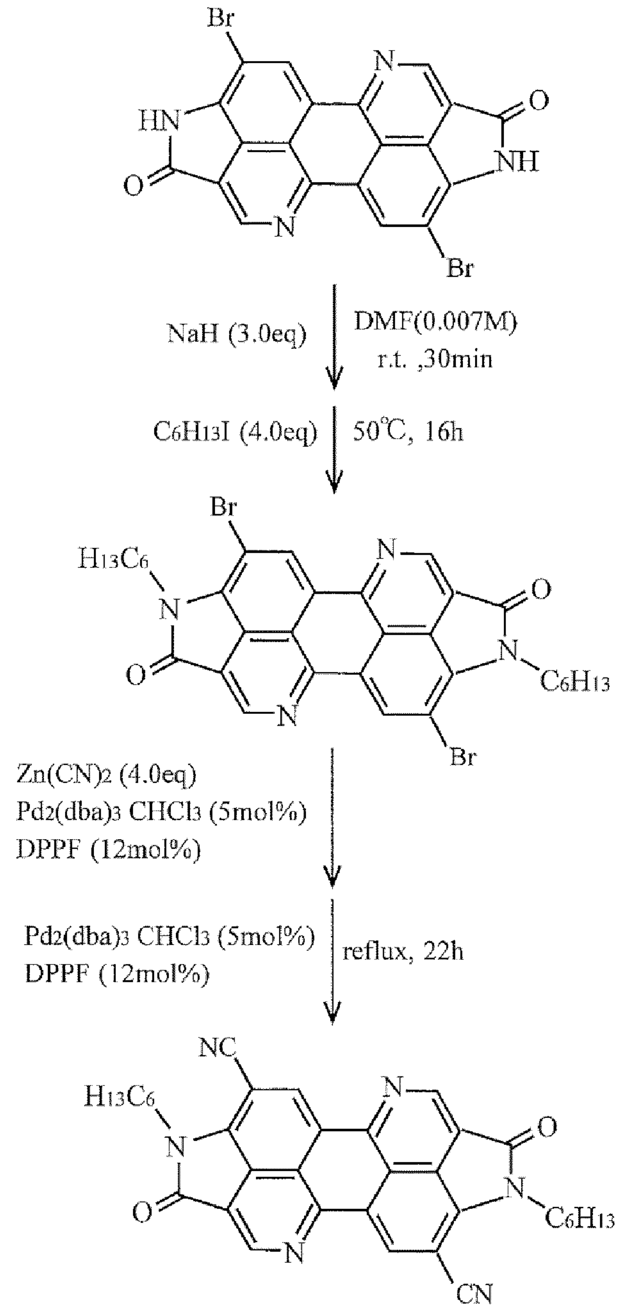


(XI)

The obtained compound (XI) and 3-phenoxytoluene were agitated at 80°C in a nitrogen atmosphere for 2 hours, and thus an organic semiconductor composition (3) having a concentration of the compound (XI) of 0.03 wt.% was obtained.

A liquid membrane with concave meniscus was formed in the same manner as in Example 6 except for using the organic semiconductor composition (3) in place of the organic semiconductor composition (1).

Under normal pressure, the substrate was heated at 100°C for 2 hours, the obtained liquid membrane was dried, and thus crystal was deposited. The glass member was then removed, and heating and drying was performed under reduced pressure ($10^{-3}$ Pa) at 100°C for 12 hours, and thus a ring-like organic semiconductor film (3) having a uniform thickness (film thickness: 50 nm) was formed on the substrate.

The obtained organic semiconductor film (3) was masked, then gold was vapor-deposited (thickness: 40 nm), and thus an organic thin film transistor (3) for measuring field effect transistor properties [gate width (W): 30 μm; gate length (L): 50 μm; and a ratio (W/L): 0.6] was obtained.

Example 9

A product was obtained by performing the following reaction, the obtained product was subjected to recrystallization using o-dichlorobenzene, and thus a compound represented by Formula (XII) below, namely a compound (XII) was obtained (6 mg). For the obtained compound, the structure was identified by $^1$H-NMR.
(400 MHz, $CDCl_2CDCl_2$ at 100°C): δ 9.49 (s, 2H), 7.44 (s, 2H), 4.34 (t, J = 7.2 Hz, 4H), 1.90 (t, J = 7.2 Hz, 4H), 1.3-1.5 (m, 20H), 0.88 (t, J = 6.8 Hz, 6H).

[Chem. 26]

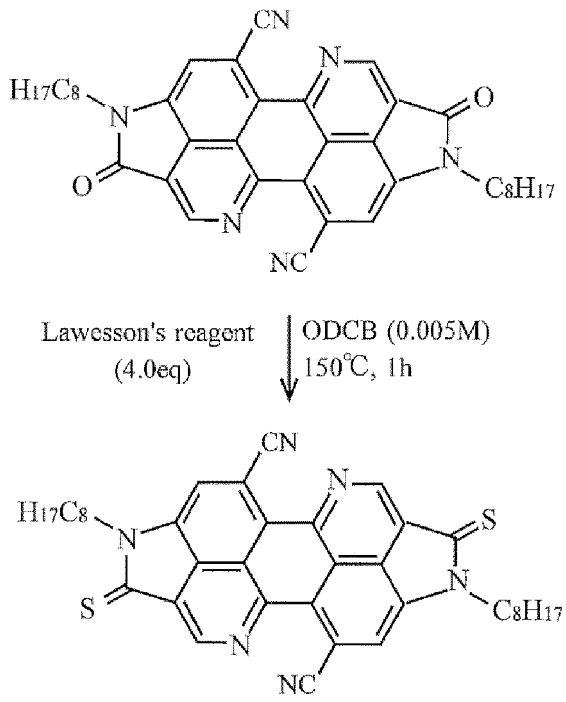


(XII)

The obtained compound (XII) and butoxybenzene were agitated at 140°C in a nitrogen atmosphere for 2 hours, and thus an organic semiconductor composition (4) having a concentration of the compound (XII) of 0.03 wt.% was obtained.
A liquid membrane with concave meniscus was formed in the same manner as in Example 6 except for using the organic semiconductor composition (4) in place of the organic semiconductor composition (1).
Under normal pressure, the substrate was heated at 100°C for 2 hours, the obtained liquid membrane was dried, and thus crystal was deposited. The glass member was then removed, and heating and drying was performed under reduced pressure ($10^{-3}$ Pa) at 160°C for 12 hours, and thus a ring-like organic semiconductor film (4) having a uniform thickness (film thickness: 50 nm) was formed on the substrate.
The obtained organic semiconductor film (4) was masked, then gold was vapor-deposited (thickness: 40 nm), and thus an organic thin film transistor (4) for measuring field effect transistor properties [gate width (W): 30 μm; gate length (L): 50 μm; and a ratio (W/L): 0.6] was obtained. The charge mobility of the obtained organic thin film transistor (4) was $3.9 \times 10^{-3}$ $cm^2$/Vs, and the threshold voltage was 1.7 V.

Example 10

The following reaction was performed. Then, the reaction was terminated by adding hydrochloric acid (1 M) to the reaction system. Then, the reaction product was filtered, and the obtained residue was washed by water, suspended in chloroform, and filtered. The obtained residue was dried, washed with a toluene/hexane mixture solution, and filtered, and thus a compound represented by Formula (XIII) below, namely a compound (XIII) was obtained (14 mg; yield: 20%). For the obtained compound, the structure was identified by $^1$H-NMR.

(400 MHz, $CDCl_2CDCl_2$ at 100°C): δ 9.23 (s, 2H), 8.75 (d, J = 7.6 Hz, 2H), 7.20 (d, J = 7.6 Hz, 2H), 4.59 (t, J = 13.6 Hz, 4H).

[Chem. 27]

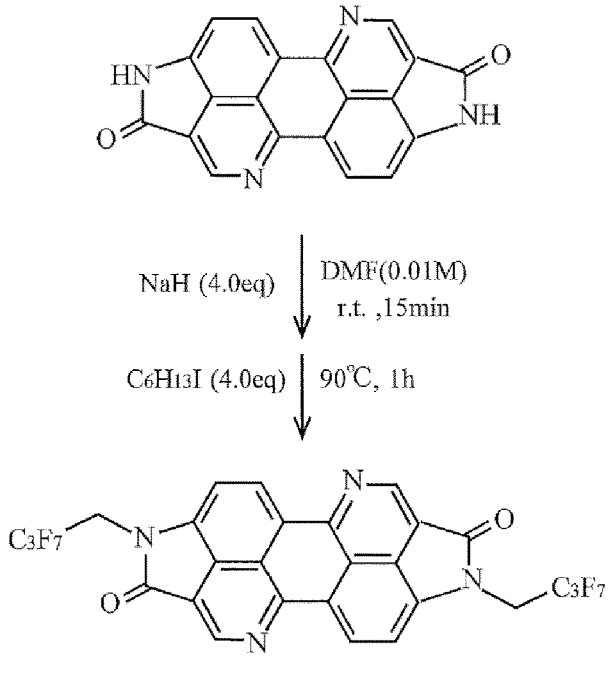


(XIII)

The obtained compound (XIII) and 1,3-dimethoxybenzene were agitated at 120°C in a nitrogen atmosphere for 1 hour, and thus an organic semiconductor composition (4) having a concentration of the compound (XIII) of 0.03 wt.% was obtained.

A liquid membrane with concave meniscus was formed in the same manner as in Example 6 except for using the organic semiconductor composition (5) in place of the organic semiconductor composition (1).

Under normal pressure, the substrate was heated at 100°C for 2 hours, the obtained liquid membrane was dried, and thus crystal was deposited. The glass member was then removed, and heating and drying was performed under reduced pressure ($10^{-3}$ Pa) at 110°C for 12 hours, and thus a ring-like organic semiconductor film (5) having a uniform thickness (film thickness: 50 nm) was formed on the substrate.

The obtained organic semiconductor film (5) was masked, then gold was vapor-deposited (thickness: 40 nm), and thus an organic thin film transistor (5) for measuring field effect transistor properties [gate width (W): 30 μm; gate length (L): 50 μm; and a ratio (W/L): 0.6] was obtained. The charge mobility in the nitrogen atmosphere of the obtained organic thin film transistor (5) was $6.57 \times 10^{-2}$ $cm^2/Vs$, and the electron effective mass ratio was 1.4. Note that the electron effective mass ratio of the compound (IX) of Comparative Example 1 was 2.8.

As a summary of the above, configurations and variations of the present disclosure are described below.

[1] A compound represented by Formula (1).

[2] A composition containing the compound represented by Formula (1) and a solvent.

[3] The composition according to [2], in which the solvent has a boiling point of 60°C or higher at normal pressure.

[4] The composition according to [2] or [3], in which a content of the solvent is from 100 to 10000 parts by weight per 1 part by weight of the compound represented by Formula (1).

[5] The composition according to any one of [2] to [4], in which a content of the compound represented by Formula (1) is 80 wt.% or greater per a total amount of non-volatile content in the composition.

[6] The composition according to any one of [2] to [5], in which a total content of the compound represented by Formula (1) and the solvent is 80 wt.% or greater per a total amount of the composition.

[7] The composition according to any one of [2] to [6], in which the compound represented by Formula (1) is at least one type of compound selected from the group consisting of compounds represented by Formulas (1-0) to (1-3).

[8] The composition according to any one of [2] to [6], in which the compound represented by Formula (1) is a compound represented by Formula (1-1) or (1-2).

[9] The composition according to any one of [2] to [6], in which the compound represented by Formula (1) is a

compound represented by Formula (1-1).

[10] The composition according to any one of [2] to [6], in which the compound represented by Formula (1) is a compound represented by Formula (2).

[11] The composition according to any one of [2] to [6], in which the compound represented by Formula (1) is at least one type of compound selected from the group consisting of compounds represented by Formulas (2-0), (2-1), (2-2a) to (2-2c), and (2-3).

[12] The composition according to any one of [2] to [6], in which the compound represented by Formula (1) is at least one type of compound selected from the group consisting of compounds represented by Formulas (2-1) and (2-2a) to (2-2c).

[13] The composition according to any one of [2] to [6], in which the compound represented by Formula (1) is a compound represented by Formula (2-1).

[14] The composition according to any one of [2] to [6], in which the compound represented by Formula (1) is a compound represented by Formula (3).

[15] The composition according to any one of [2] to [6], in which the compound represented by Formula (1) is at least one type of compound selected from the group consisting of compounds represented by Formulas (3-0) to (3-3).

[16] The composition according to any one of [2] to [6], in which the compound represented by Formula (1) is a compound represented by Formula (3-1) or (3-2).

[17] The composition according to any one of [2] to [6], in which the compound represented by Formula (1) is a compound represented by Formula (3-1).

[18] The composition according to any one of [2] to [6], in which the compound represented by Formula (1) is a compound represented by Formula (4).

[19] The composition according to any one of [2] to [6], in which the compound represented by Formula (1) is at least one type of compound selected from the group consisting of compounds represented by Formulas (4-0), (4-1), (4-2a) to (4-2c), and (4-3).

[20] The composition according to any one of [2] to [6], in which the compound represented by Formula (1) is at least one type of compound selected from the group consisting of compounds represented by Formulas (4-1) and (4-2a) to (4-2c).

[21] The composition according to any one of [2] to [6], in which the compound represented by Formula (1) is a compound represented by Formula (4-1).

[22] The composition according to any one of [2] to [6], in which the compound represented by Formula (1) is a compound represented by Formula (2-1-1).

[23] The composition according to any one of [2] to [6], in which the compound represented by Formula (1) is a compound represented by Formula (2-1-2).

[24] The composition according to any one of [2] to [6], in which the compound represented by Formula (1) is a compound represented by Formula (4-1-1).

[25] The composition according to any one of [2] to [6], in which the compound represented by Formula (1) is a compound represented by Formula (4-1-2).

[26] The composition according to any one of [1] to [25], in which $R^1$ in the formula is a monovalent hydrocarbon group or a monovalent halogenated hydrocarbon group.

[27] The composition according to any one of [1] to [25], in which $Y^1$ in the formula represents a sulfur atom.

[28] The composition according to any one of [1] to [25], in which $R^1$ in the formula represents a monovalent hydrocarbon group or a monovalent halogenated hydrocarbon group, and $Y^1$ represents a sulfur atom.

[29] The composition according to any one of [2] to [28], which is an ink for organic semiconductor film formation.

[30] Use of the composition according to any one of [2] to [28] as an ink for organic semiconductor film formation.

[31] A method for producing an organic semiconductor film, the method including applying a solvent-dissolved material containing the compound represented by Formula (1) on a substrate and drying to form a film containing the compound.

[32] A method for producing an organic semiconductor film, the method including applying the composition according to any one of [2] to [28] on a substrate and drying to forming a film containing the compound.

[33] A method for producing an organic semiconductor device, the method including applying a solvent-dissolved material of the compound represented by Formula (1) on a substrate and drying to form a film containing the compound.

[34] A method for producing an organic semiconductor device, the method including applying the composition according to any one of [2] to [28] on a substrate and drying to form a film containing the compound.

[35] An organic semiconductor film containing a compound represented by Formula (1).

[36] An organic semiconductor film containing a solidified material of the composition according to any one of [2] to [28].

[37] The organic semiconductor film according to [35] or [36], having charge mobility of $10^{-5}$ $cm^2/Vs$ or greater.

[38] An organic semiconductor device including the organic semiconductor film according to any one of [35] to [37].
[39] The organic semiconductor device according to [38], which is a switching element, a diode, a transistor, a photoelectric conversion device, a solar cell, or a photoelectric conversion element.

Industrial Applicability

By using the compound (1), an organic semiconductor device, such as organic thin film transistors, organic thin film solar cells, and photoelectric conversion elements, that is thin and light-weighted and that has flexibility, high conductivity, and high stability can be produced by a printing process.

## Claims

1. A compound represented by Formula (1):

[Chem. 1]

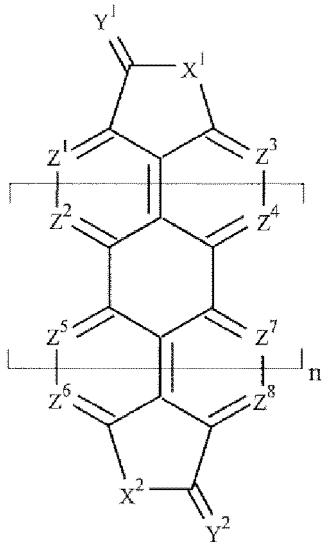

(1)

where in Formula (1), $X^1$ and $X^2$ are the same or different and each represent -O-, $-NR^1-$, or $-PR^2-$; $Y^1$ and $Y^2$ are the same or different and each represent an oxygen atom or a sulfur atom; $Z^1$ to $Z^8$ are the same or different and each represent =N- or $=CR^3-$; the $R^1$, $R^2$, and $R^3$ are the same or different and each represent a hydrogen atom or an organic group; and n represents an integer of 0 or greater.

2. An organic semiconductor composition comprising the compound according to claim 1 and a solvent.

3. A method for producing an organic semiconductor film, the method comprising applying a solvent-dissolved material containing the compound according to claim 1 on a substrate and drying to form a film containing the compound.

4. A method for producing an organic semiconductor device, the method comprising applying a solvent-dissolved material containing the compound according to claim 1 on a substrate and drying to form a film containing the compound.

5. An organic semiconductor film comprising a compound represented by Formula (1) below:

[Chem. 2]

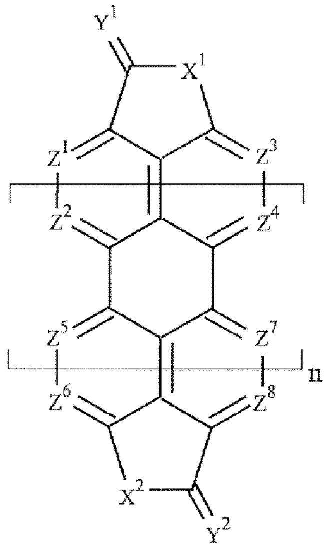

(1)

where in Formula (1), $X^1$ and $X^2$ are the same or different and each represent -O-, $-NR^1-$, or $-PR^2-$; $Y^1$ and $Y^2$ are the same or different and each represent an oxygen atom or a sulfur atom; $Z^1$ to $Z^8$ are the same or different and each represent =N- or $=CR^3-$; the $R^1$, $R^2$, and $R^3$ are the same or different and each represent a hydrogen atom or an organic group; and n represents an integer of 0 or greater.

**6.** An organic semiconductor device comprising the organic semiconductor film according to claim 5.

**INTERNATIONAL SEARCH REPORT**

International application No.
**PCT/JP2021/032043**

**A. CLASSIFICATION OF SUBJECT MATTER**

***H01L 51/30***(2006.01)i; ***C07D 471/22***(2006.01)i; ***H01L 29/786***(2006.01)i; ***H01L 51/05***(2006.01)i; ***H01L 51/46***(2006.01)i
FI: H01L29/28 250H; H01L29/28 100A; H01L29/78 618B; H01L31/04 154C; H01L31/04 154D; C07D471/22 CSP

According to International Patent Classification (IPC) or to both national classification and IPC

**B. FIELDS SEARCHED**

Minimum documentation searched (classification system followed by classification symbols)

H01L51/30; C07D471/22; H01L29/786; H01L51/05; H01L51/46

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | US 2016/0240789 A1 (POLYERA CORPORATION) 18 August 2016 (2016-08-18) paragraphs [0034]-[0058], fig. 1 | 1-6 |
| X | DE 1156528 B1 (BADISCHE ANILIN- & SODA-FABRIK AKTIENGESELLSCHAFT) 31 October 1963 (1963-10-31) column 7, line 58 to column 8, line 24 | 1 |
| X | ECKSTEIN, J. Brian et al. Naphthalene bis(4,8-diamino-1,5-dicarboxyl)amide Building Block for Semiconducting Polymers. Journal of the American Chemical Society. 26 September 2017, vol. 139, issue 41, pp. 14356-14359, DOI:10.1021/jacs.7b07750 fig. 3A | 1 |
| A | JP 2007-523070 A (WELLA AKTIENGESELLSCHAFT) 16 August 2007 (2007-08-16) | 1-6 |
| A | JP 2018-006745 A (FUJIFILM CORPORATION) 11 January 2018 (2018-01-11) | 1-6 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 November 2021** | **16 November 2021** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2021/032043**

| Patent document cited in search report | Publication date (day/month/year) | Patent family member(s) | Publication date (day/month/year) |
|---|---|---|---|
| US 2016/0240789 A1 | 18 August 2016 | (Family: none) | |
| DE 1156528 B1 | 31 October 1963 | (Family: none) | |
| JP 2007-523070 A | 16 August 2007 | US 2007/0157398 A1 | |
| | | WO 2005/075481 A1 | |
| | | EP 1730149 A1 | |
| | | DE 102004006143 A1 | |
| | | BR PI0418509 A | |
| JP 2018-006745 A | 11 January 2018 | US 2019/0131546 A1 | |
| | | WO 2018/003701 A1 | |
| | | EP 3476845 A1 | |
| | | TW 201829410 A | |
| | | CN 109478595 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020147355 A
- WO 2011082234 A
- JP 2019178160 A